(19) Europäisches Patentamt
European Patent Office
Office européen des brevets

(11) EP 3 035 043 A1

(12) EUROPÄISCHE PATENTANMELDUNG

(43) Veröffentlichungstag:
22.06.2016  Patentblatt 2016/25

(51) Int Cl.:
G01N 27/22 (2006.01)   G01F 23/26 (2006.01)

(21) Anmeldenummer: 14198966.5

(22) Anmeldetag: 18.12.2014

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(71) Anmelder: Airbus Defence and Space GmbH
85521 Ottobrunn (DE)

(72) Erfinder:
• Legner, Wolfgang
85635 H.-Siegertsbrunn (DE)
• Fruedberger, Alois
85667 Oberpframmern (DE)

(74) Vertreter: Kastel, Stefan et al
Kastel Patentanwälte
St.-Cajetan-Straße 41
81699 München (DE)

(54) **Sensoreinrichtung und Herstellverfahren hierfür sowie Messvorrichtung**

(57) Die Erfindung betrifft eine Sensoreinrichtung (28, 200, 200') zum Erfassen wenigstens eines Materialparameters eines ein Matrixmaterial (24) aufweisenden Verbundwerkstoffes, umfassend eine Schichtstruktur (66) mit einem darin vorgesehenen Probenraum (38) zum Aufnehmen des Matrixmaterials (24), mit einer durch elektrisch leitende Schichten (68, 70) gebildeten Felderzeugungsanordnung (64) zum Erzeugen eines elektrischen Feldes in dem Probenraum (38) und mit einer die elektrisch leitenden Schichten (68, 70) der Felderzeugungsanordnung (64) voneinander isolierenden Isolierschicht (72).

Fig. 11

**Beschreibung**

Die Erfindung betrifft eine Sensoreinrichtung zum Erfassen eines Materialparameters eines ein Matrixmaterial aufweisenden Verbundwerkstoffs. Ferner betrifft die Erfindung ein Herstellverfahren für eine solche Sensoreinrichtung und eine Messvorrichtung mit mehreren solchen Sensoreinrichtungen.

Aus Sernek und Kamke, Int. J. Adhes. Adhes., Vol. 27, no. 7, pp. 562-567, 2007 ist ein Verfahren und einen Kammsensor zur Überwachung eines Aushärtungsprozesses eines Phenolformaldehyd-Klebstoffes bekannt.

Kazilas und Partridge, Poymer, Vol. 46, no. 16, pp. 5868-5878 beschreiben ein Modell für einen Zusammenhang von Aushärtungstemperatur, Aushärtungsgrad und elektrischer Impedanz von Kammsensoren.

Hardis et al., Compos. Part Appl. Sci. Manuf., Vol 49, pp. 100-108, 2013 beschreiben einen Plattenkondensator zur Untersuchung der Aushärtungskinetik von Epoxidharz.

Die bekannten Sensoren erlauben eine punktuelle Messung des Verbundwerkstoffes. Zusätzliche Sensoren können die Messfläche vergrößern, sind jedoch mit hohem Aufwand und weiteren Kosten verbunden.

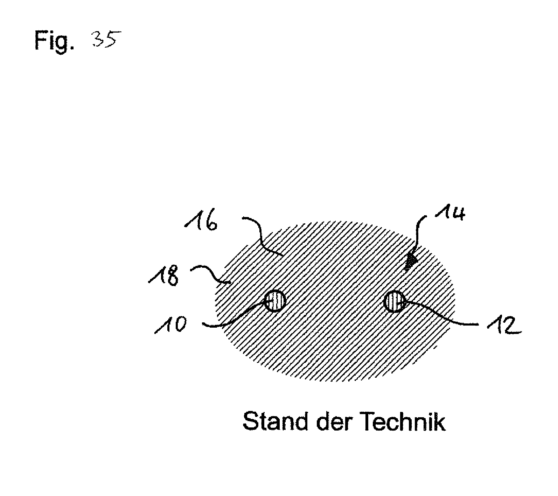

Eine Zweidrahtleitung, wie in Fig. 35 beispielhaft wiedergegeben, wurde in Dominauskas et al., Compos. Part Appl. Sci. Manuf., Vol. 34, pp. 67- 74, 2003 und Vol. 38, pp. 138-146, 2007 zur Überwachung eines Herstellungsverfahrens von Verbundwerkstoffen beschrieben.

Eine erste Leitung 10 wird an Erde angeschlossen, während eine zweite Leitung 12 mit einem Spannungsimpuls beaufschlagt wird. Die Leitungen 10, 12 werden in einem Dielektrikum 14 angeordnet. Das Dielektrikum 14 enthält ein Fasermaterial 16 und wird mit einem Harz 18 infusioniert. Durch Zeitbereichsreflektometrie lässt sich die Position der Fließfront des Harzes 18 und dessen Aushärtungsgrad/Vernetzungsgrad bestimmen. Derartige Sensoren umfassen zwei parallel angeordnete Leiterbahnen. Daher können elektrisch leitende Materialien (beispielsweise leitende Kohlenstofffasern, metallische Formwerkzeuge,...), die sich in der Nähe des Sensors befinden, das elektrische Feld der Sensoren zusätzlich beeinflussen und so eine präzise Messung zumindest erschweren. Mit der in Fig. 35 dargestellten Konfiguration können daher lediglich für nicht leitende Materialien, wie etwa Fasern, und nicht leitende Formwerkzeuge gute Ergebnisse erzielt werden.

Der Erfindung liegt die Aufgabe zugrunde, eine einfach und kostengünstig in Serie produzierbare Sensoreinrichtung zum präzisen Messen von Materialparametern zu schaffen. Außerdem sollen ein vorteilhaftes Herstellverfahren sowie vorteilhafte Verwendungen angegeben werden.

Als Lösung wird eine Sensoreinrichtung gemäß Anspruch 1 sowie ein Herstellverfahren hierfür sowie eine Messanordnung und ein Materialparametererfassungsverfahren gemäß der weiteren unabhängigen Ansprüche vorgeschlagen.

Vorteilhafte Ausgestaltungen sind Gegenstand der Unteransprüche.

Gemäß einem Aspekt schafft die Erfindung eine Sensoreinrichtung zum Erfassen wenigstens eines Materialparameters eines ein Matrixmaterial aufweisenden Verbundwerkstoffes, umfassend eine Schichtstruktur mit einem darin vorgesehenen Probenraum zum Aufnehmen des Matrixmaterials, mit einer durch elektrisch leitende Schichten gebildeten Felderzeugungsanordnung zum Erzeugen eines elektrischen Feldes in dem Probenraum und mit einer die elektrisch leitenden Schichten der Felderzeugungsanordnung voneinander isolierenden Isolierschicht.

Es ist bevorzugt, dass die Schichtstruktur eine den Probenraum und/oder wenigstens einen Teil der Felderzeugungsanordnung elektrisch abschirmende durch wenigstens eine elektrische leitende Schicht gebildete Abschirmung aufweist.

Es ist bevorzugt, dass die Felderzeugungsanordnung eine durch wenigstens eine elektrisch leitende Schicht gebildete erste Elektrode und eine durch wenigstens eine elektrisch leitende Schicht gebildete zweite Elektrode zum Erzeugen des elektrischen Feldes aufweist.

Es ist bevorzugt, dass die Felderzeugungsanordnung wenigstens eine durch wenigstens eine elektrisch leitende Schicht der Schichtstruktur gebildete Innenelektrode und eine durch wenigstens eine elektrisch leitende Schicht der Schichtstruktur gebildete die wenigstens eine Innenelektrode abschirmende Außenelektrode aufweist.

Es ist bevorzugt, dass die Felderzeugungsanordnung eine durch wenigstens eine elektrisch leitende Schicht der Schichtstruktur gebildete erste Innenelektrode und eine durch wenigstens eine elektrische leitende Schicht gebildete zweite Innenelektrode aufweist.

Es ist bevorzugt, dass die Sensoreinrichtung sich flächenartig und/oder in einer Längsrichtung erstreckt und/oder ein Verhältnis von Länge zu Breite der Sensoreinrichtung von mindestens 5:1 aufweist.

Es ist bevorzugt, dass die Schichtstruktur eine elektrisch leitende Messschicht aufweist. Es ist bevorzugt, dass die Schichtstruktur eine elektrisch leitende Erdungsschicht zum Anlegen an Masse aufweist. Es ist bevorzugt, dass die Schichtstruktur eine elektrisch leitende Zwischenerdungsschicht, die an derselben Seite der Isolierschicht wie die Messschicht angeordnet ist aufweist. Es ist bevorzugt, dass die Schichtstruktur eine elektrisch isolierende Zwischenisolierschicht, die an die Isolierschicht und/oder die Messschicht und/oder die Zwischenerdungsschicht und/oder den Probenraum angrenzt aufweist. Es ist bevorzugt, dass die Schichtstruktur eine elektrisch leitende Deckschicht, die an die Zwischenisolierschicht angrenzt, so dass Zwischenisolierschicht die Deckschicht von der Messschicht isoliert aufweist.

Es ist bevorzugt, dass die Schichtstruktur eine Verbindungsschicht zum Verbinden von gesondert hergestellten Schichtstrukturmodulen, um so die Schichtstruktur zu bilden aufweist. Es ist bevorzugt, dass die Schichtstruktur eine Isolierschicht aus einem Lackmaterial, durch Spin-Coating aufgetragenem Material, aufgespraytem Material, durch Siebdruck aufgetragenem Material und/oder durch PVD aufgetragenem Material aufweist.

Es ist bevorzugt, dass sich die Felderzeugungsanordnung über eine Großteil der Länge der Schichtstruktur erstreckt.

Es ist bevorzugt, dass die Schichtstruktur einen länglichen Durchlass oder eine Reihe von Durchlässen zum Durchlassen von zu messenden Material in den länglichen Probenraum aufweist.

Es ist bevorzugt, dass eine Durchlassabmessung des wenigstens einen Durchlasses entsprechend einer Kapazitätsmessfrequenz ausgebildet ist und/oder dass der wenigstens eine Durchlass als eine sich im Wesentlichen in Längsrichtung erstreckende Öffnung oder dass eine Reihe von im Wesentlichen zylinderförmigen Öffnungen vorgesehen sind und/oder dass der Durchlass auf lediglich einer Seite der Schichtstruktur vorgesehen ist.

Es ist bevorzugt, dass die Schichtstruktur spiegelsymmetrisch bezüglich einer Längsmittelebene oder bezüglich zwei Längsmittelebenen ausgebildet ist.

Gemäß einem weiteren Aspekt schafft die Erfindung eine Messvorrichtung zum Messen wenigstens eines Materialparameters eines ein Matrixmaterial aufweisenden Verbundwerkstoffes entlang einer Messstrecke, mit einem sich entlang der Messstrecke erstreckenden Bauteil, an dem mehrere Sensoreinrichtungen nach einer der vorstehenden Ausgestaltungen an zumindest teilweise unterschiedlichen Bereichen der Messstrecke ausgebildet sind.

Vorzugsweise ist eine Schnittstelle vorgesehen, die zum gemeinsamen elektrischen und/oder drahtlosen Anschließen von wenigstens zwei der mehreren Sensoreinrichtungen an die Materialparametererfassungsvorrichtung ausgebildet ist.

Gemäß einem weiteren Aspekt schafft die Erfindung ein Verfahren zum Herstellen einer Sensoreinrichtung zum Messen von Materialparameter durch Messen von dielektrischen Materialeigenschaften mit den Schritten:

a) Bereitstellen wenigstens eines Schichtstrukturrohlings mit einer elektrisch isolierenden Isolierschicht und mit einer elektrisch leitenden Schicht und

b) Ausbilden von Elektroden einer Felderzeugungsanordnung und/oder einer Abschirmung und/oder des Probenraums durch Materialstrukturierung, Materialauftrag und/oder Materialabtrag an dem wenigstens einen Schichtstrukturrohling.

Eine bevorzugte Ausgestaltung ist des Verfahrens ist gekennzeichnet durch:

c) Bilden von mehreren Schichtstrukturmodulen aus mehreren Schichtstrukturrohlingen und

d) Verbinden der Schichtstrukturmodule zu einer den Probenraum und die Felderzeugungsanordnung und eine Abschirmung aufweisenden Schichtstruktur.

Weiter betrifft die Erfindung die Verwendung einer Sensoreinrichtung nach einer der zuvor erläuterten Ausgestaltungen oder herstellbar nach einem der zuvor erläuterten Verfahren, zum Messen einer Fließfront und/oder eines Aushärtungsgrades und/oder eines Vernetzungsgrades, insbesondere des Matrixmaterials, beim Herstellen von faserverstärkten Verbundbauteilen.

Es ist bevorzugt, dass die Sensoreinrichtung in ein Werkzeug und/oder in einem Vakuumsack und/oder auf einem Vakuumsack und/oder in dem Verbundbauteil eingebracht ist.

Vorzugsweise dient die Sensoreinrichtung zum Erfassen wenigstens eines Materialparameters eines Verbundwerkstoffs, der ein Matrixmaterial aufweist, und ist vorzugsweise mit einem Probenraum versehen, der zwischen einer Innenelektrode und einer die Innenelektrode im Querschnitt umgebenden Außenelektrode angeordnet und zum Aufnehmen des Matrixmaterials und/oder des Verbundwerkstoffs ausgebildet ist.

Vorzugsweise umfasst die Sensoreinrichtung eine erste Innenelektrode und eine zweite Innenelektrode. Vorzugsweise ist die zweite Innenelektrode in dem Probenraum angeordnet und insbesondere im Querschnitt von der Außenelektrode umgeben.

Es ist bevorzugt, dass die Außenelektrode Öffnungen und/oder Poren aufweist. Die Öffnungen und/oder die Poren sind bevorzugt geeignet, ein Eindringen des Matrixmaterials in den Probenbraum zu gestatten.

Es ist bevorzugt, dass die Außenelektrode ein leitendes Material aufweist. Es ist bevorzugt, dass die Außenelektrode eine Konsolidierungseinrichtung zum Aushärten des Verbundwerkstoffs aufweist.

Es ist bevorzugt, dass die Innenelektrode und/oder die Außenelektrode aus Metall und/oder Kupfer und/oder Stahl gefertigt sind.

Es ist bevorzugt, dass der Probenraum zum Aufnehmen eines Fasermaterials und/oder eines Prepregs und/oder eines Matrixmaterials und/oder des Verbundwerkstoffs ausgebildet ist.

Gemäß einem weiteren Aspekt schafft die Erfindung eine Messanordnung zum Ermitteln wenigstens eines Materialparameters eines Verbundwerkstoffs mit einer, insbesondere hierin beschriebenen, Sensoreinrichtung wobei der Probenraum ein Dielektrikum aufweist, das teilweise oder vollständig aus einem Fasermaterial und einem Matrixmaterial und/oder einem Prepreg und/oder dem Verbundwerkstoff gebildet ist.

Gemäß einem weiteren Aspekt schafft die Erfindung eine Messanordnung zum Ermitteln wenigstens eines Materialparameters eines Verbundwerkstoffs mit einer, insbesondere hierin beschriebenen, Materialparameterermittlungsvorrichtung, wobei der Probenraum ein Dielektrikum aufweist, das teilweise oder vollständig aus einem Fasermaterial und/oder einem Matrixmaterial und/oder einem Prepreg und/oder dem Verbundwerkstoff gebildet ist und/oder das in der axialen Richtung der Sensoreinrichtung bewegbar ist.

Es ist bevorzugt, dass der Verbundwerkstoff aus einem Fasermaterial, insbesondere einem Fasergewebe und/oder Fasergelege, und einem Matrixmaterial und/oder einem Prepreg gebildet ist.

Es ist bevorzugt, dass das Fasermaterial Glasfasern aufweist.

Es ist bevorzugt, dass das Fasermaterial Kohlenstofffasern aufweist.

Es ist bevorzugt, dass das Fasermaterial Aramidfasern aufweist.

Es ist bevorzugt, dass das Prepreg ein Fasergewebe aufweist.

Es ist bevorzugt, dass das Prepreg ein Fasergelege aufweist. Es ist bevorzugt, dass das Prepreg Glasfasern aufweist.

Es ist bevorzugt, dass das Prepreg Kohlenstofffasern aufweist.

Es ist bevorzugt, dass das Prepreg Aramidfasern aufweist.

Es ist bevorzugt, dass das Matrixmaterial ein Harz enthält. Es ist bevorzugt, dass das Matrixmaterial ein Kunstharz enthält.

Die Sensoreinrichtung wird bevorzugt verwendet in einem Materialparametererfassungsverfahren zum Erfassen wenigstens eines Materialparameters eines Verbundwerkstoffs, gekennzeichnet durch die Schritte:

1 Anordnen einer ersten Elektrode und einer zweiten Elektrode in oder an dem Verbundwerkstoff;

2 Einspeisen eines ersten Detektionsspannungsimpulses mit einer ersten Detektionsspannungsimpulsamplitude in die erste Elektrode;

3 Einspeisen eines zweiten Detektionsspannungsimpulses mit einer zweiten Detektionsspannungsimpulsamplitude in die zweite Elektrode.

4 Erfassen einer Reflexionsspannungsimpulslaufzeit und/oder einer Reflexionsspannungsimpulsamplitude eines aus den Detektionsspannungsimpulsen hervorgegangenen Reflexionsspannungsimpulses;

5 Ermitteln des Materialparameters aus der Reflexionsspannungsimpulslaufzeit und/oder der Reflexionsspannungsimpulsamplitude.

Es ist bevorzugt, dass die erste Elektrode als Innenelektrode ausgebildet ist. Es ist bevorzugt, dass die zweite Elektrode als Außenelektrode ausgebildet ist. Es ist bevorzugt, dass der Verbundwerkstoff und/oder das Matrixmaterial in einem zwischen einer Innenelektrode und einer die Innenelektrode im Querschnitt wenigstens teilweise oder vollständig umgebenden Außenelektrode vorgesehenen Probenraum angeordnet wird.

Vorzugsweise umfasst das Verfahren einen der Schritte:

6 Anschließen der ersten Elektrode oder der zweiten Elektrode an Erde;

7 Anschließen der Außenelektrode an Erde.

Vorzugsweise wird der Schritt 2 simultan/gleichzeitig mit Schritt 3 durchgeführt.

Es ist bevorzugt, dass die zweite Detektionsspannungsimpulsamplitude betragsmäßig gleich der ersten Detektionsspannungsimpulsamplitude ist.

Es ist bevorzugt, dass die zweite Detektionsspannungsimpulsamplitude das umgekehrte Vorzeichen der ersten Detektionsspannungsimpulsamplitude aufweist.

Es ist bevorzugt, dass Schritt 1 das Bewegen des Matrixmaterial durch den Probenraum, insbesondere entlang der axialen Richtung des Probenraums, enthält.

Es ist bevorzugt, dass Schritt 1 das Relativbewegen des Matrixmaterials relativ zu der Innenelektrode, insbesondere entlang der axialen Richtung der Innenelektrode enthält.

Es ist bevorzugt, dass Schritt 1 das Relativbewegen des Matrixmaterials relativ zu der Außenelektrode, insbesondere entlang der axialen Richtung der Außenelektrode enthält.

Vorzugsweise wird das Verfahren durch Verwenden einer hierin beschriebenen Sensoreinrichtung durchgeführt.

Vorzugsweise wird das Verfahren durch Verwenden einer hierin beschriebenen Messanordnung durchgeführt.

Vorzugsweise wird das Verfahren durch Verwenden einer hierin beschriebenen Materialparametererfassungsvorrichtung durchgeführt.

Gemäß einem weiteren Aspekt schafft die Erfindung eine Materialparameterermittlungsvorrichtung zum Ermitteln wenigstens eines Materialparameters eines Verbundwerkstoffs, mit einer Sensoreinrichtung gemäß einer der zuvor beschriebenen Ausgestaltungen, die zum Erfassen von physikalischen Parametern ausgebildet ist und die einen zwischen einer Innenelektrode und einer die Innenelektrode im Querschnitt umgebenden Außenelektrode vorgesehenen Probenraum zum Aufnehmen des Verbundwerkstoffs aufweist, und mit einer Steuereinrichtung, die zum Steuern der Sensoreinrichtung ausgebildet ist.

Es ist bevorzugt, dass die Steuereinrichtung ein Spannungsimpulserzeugungsmodul aufweist, das zum Erzeugen und Einspeisen wenigstens eines Spannungsimpulses mit einer vorbestimmen Spannungsimpulsamplitude ausgebildet ist.

Es ist bevorzugt, dass die Steuereinrichtung ein Spannungsimpulserfassungsmodul aufweist, das zum Erfassen einer Spannungsimpulsamplitude eines Spannungsimpulses ausgebildet ist.

Es ist bevorzugt, dass die Steuereinrichtung ein Spannungsimpulslaufzeiterfassungsmodul aufweist, das zum Erfassen einer Spannungsimpulslaufzeit ausgebildet ist.

Es ist bevorzugt, dass die Steuereinrichtung ein Auswertemodul aufweist, das zum Ermitteln eines Materialparameters des Verbundwerkstoffs aus einer Spannungsimpulsamplitude und/oder einer Spannungsimpulslaufzeit ausgebildet ist.

Es ist bevorzugt, dass das Spannungsimpulserzeugungsmodul ausgebildet ist, die Innenelektrode mit einem ersten Detektionsspannungsimpuls mit einer ersten Detektionsspannungsimpulsamplitude zu beaufschlagen.

Es ist bevorzugt, dass das Spannungsimpulserzeugungsmodul ausgebildet ist, die Außenelektrode mit einem zweiten Detektionsspannungsimpuls mit einer zweiten Detektionsspannungsimpulsamplitude zu beaufschlagen.

Es ist bevorzugt, dass das Spannungsimpulserfassungsmodul zum Erfassen einer Reflexionsspannungsimpulsamplitude eines aus einem Detektionsspannungsimpuls hervorgegangenen Reflexionsspannungsimpulses ausgebildet ist.

Es ist bevorzugt, dass das Spannungsimpulslaufzeiterfassungsmodul zum Erfassen einer Reflexionsspannungsimpulslaufzeit eines aus einem Detektionsspannungsimpuls hervorgegangenen Reflexionsspannungsimpulses ausgebildet ist.

Es ist bevorzugt, dass das Auswertemodul zum Ermitteln einer dielektrischen Permeabilität ausgebildet ist

Es ist bevorzugt, dass das Auswertemodul zum Ermitteln einer Position eines in dem Probenraum angeordneten Dielektrikums ausgebildet ist.

Ausführungsbeispiele der Erfindung werden im Folgenden anhand der beigefügten schematischen Zeichnungen näher erläutert. Darin zeigen:

| | |
|---|---|
| Fig. 1 | eine schematische Ansicht eines Ausführungsbeispiels einer Materialparametererfassungsvorrichtung; |
| Fig. 2 | eine schematische Ansicht eines weiteren Ausführungsbeispiels einer Materialparametererfassungsvorrichtung; |
| Fig. 3, 4 | schematische Prinzipdarstellungen einer ersten Ausführungsform einer in den Materialparametererfassungsvorrichtungen einzusetzenden Sensoreinrichtung zur Verdeutlichung des Wirkungsprinzips davon; |
| Fig. 5 | eine schematische stark vereinfacht wiedergegebene Querschnittsansicht eines Modells einer zweiten Ausführungsform einer in den Materialparametererfassungsvorrichtungen der Fig. 1 und 2 einzusetzenden Sensoreinrichtung zur Verdeutlichung des Wirkungsprinzips davon; |
| Fig. 6 | eine schematische Ansicht eines Ausführungsbeispiels einer Verbindungsanordnung für die Sensoreinrichtung; |
| Fig. 7 | ein Spannungs-Zeit-Diagramm einer single-ended und einer differentiellen TDR-Messung; |
| Fig. 8 | ein weiteres Spannungs-Zeit-Diagramm einer differentiellen TDR-Messung; |
| Fig. 9A bis Fig. 9C | eine schematische Darstellung einer single-ended TDR-Messung; |
| Fig. 10A bis Fig. 10C | eine schematische Darstellung einer differentiellen TDR-Messung; |
| Fig. 11 | eine Perspektivansicht eines praktischen Ausführungsbeispiels der Sensoreinrichtung; |
| Fig. 12 | eine Draufsicht auf die Sensoreinrichtung gemäß Fig. 11; |
| Fig. 13 | einen Schnitt durch die Sensoreinrichtung entlang der Linie C-C aus Fig. 12; |

Fig. 14 bis Fig. 18    Verfahrensschritte eines Ausführungsbeispiels eines Herstellverfahrens der Sensoreinrichtung;

Fig. 19 bis Fig. 23    Verfahrensschritte eines weiteren Ausführungsbeispiels eines Herstellverfahrens der Sensoreinrichtung;

Fig. 24 bis Fig. 27    Verfahrensschritte eines weiteren Ausführungsbeispiels eines Herstellverfahrens der Sensoreinrichtung;

Fig. 28    eine Alternative zu dem Herstellverfahren der Fig. 24 bis 27;

Fig. 29    eine weitere Alternative der wie bei Fig. 28 herstellbaren Sensoreinrichtung;

Fig. 30    eine Variante der Sensoreinrichtung von Fig. 29;

Fig. 31-33    Beispiele für Verschaltungen der durch die unterschiedlichen Herstellverfahren erhältlichen Sensoreinrichtungen;

Fig. 34    ein Beispiel für eine Messvorrichtung; und

Fig. 35    eine Sensoreinrichtung nach dem Stand der Technik.

Die im Folgenden anhand der Figuren näher erläuterten dielektrischen Sensoreinrichtungen 28, 200 stellen eine wirkungsvolle Methode zur Überwachung der Fließfront und des Aushärtegrades bei Infusionsprozessen zum Herstellen von Verbundbauteilen, beispielsweise Faserverbundbauteilen, dar. Zum Überwachen größerer Flächen eignen sich vor allem längliche Sensoren, die etwa über Zeitbereichsreflektometrie ausgewertet werden können. Zeitbereichsreflektometrie wird auch Time-Domain-Reflectometry oder kurz TDR genannt.

Insbesondere wird ein vorzugsweise länglicher Probenraum 38 geschaffen, in oder an dem wenigstens eine Innenelektrode 30 vorgesehen ist und der durch eine Außenelektrode 34 abschirmbar ist. Dadurch entsteht ein Probenraum 38, der gegenüber Störfeldern aus der Umgebung abgeschirmt ist. Der Probenraum 38 wird durch darin befindliches zu messendes Material, wie z.B. Verbundwerkstoff oder dessen Matrixmaterial, etwa Harz, beeinflusst, und somit wird eine Messung ermöglicht. Beim Anlegen einer Spannung zwischen der Innenelektrode 30 und der Außenelektrode 34 bildet sich ein elektrisches Feld aus, das gegenüber der Umgebung geschirmt ist. Die dielektrischen Eigenschaften des isolierenden Mediums (Dielektrikum) zwischen Innenelektrode 32 und Außenelektrode 34 bestimmen die Eigenschaften des Sensors. Beispielsweise kann der Wellenwiderstand der Leitung mit einer geeigneten Messmethode, wie etwa TDR, gemessen werden.

Der durch die Sensoreinrichtung 28 gebildete Sensor ist so aufgebaut, dass die Innenelektrode 32 und die Außenelektrode 34 durch ein dielektrisches oder elektrisch nicht leitendes Material isoliert sind, das Poren oder Öffnungen aufweist, Der die Außenelektrode 34 bildende Außenleiter ist ebenfalls mit Öffnungen oder Poren ausgebildet. Ein flüssiges Medium, etwa Harz während des Infusionierens, kann so Material tränken und/oder in den Probenraum 38 eintreten. Durch die sich verändernden Eigenschaften des Dielektrikums in dem Probenraum 38 können beispielsweise die Position der Fließfront des Harzes oder eine Veränderung der dielektrischen Permittivität während der Aushärtung des Harzes ermittelt werden.

Durch die Schirmung des elektrischen Feldes ist der Sensor vergleichsweise unempfindlich gegenüber äußeren Einflüssen, wie etwa metallischen Werkzeugen oder leitenden Fasern. Somit kann die Präzision und/oder Qualität der Messung, insbesondere verglichen mit Zweidraht- oder Kammsensoren, verbessert werden.

Die Messung kann "single-ended" erfolgen, d.h. lediglich ein Leiter des Sensors wird mit einer Spannung beaufschlagt, während der andere Leiter als Erde dient. Zur weiteren Verbesserung des Signal-zu-Rausch-Abstandes kann die sogenannte differentielle TDR verwendet werden. Dabei werden beide Leiter des Sensors mit einem gegenpoligen Signal beaufschlagt. Bei gleichbleibender differentieller Signalamplitude können so Reflexionen an Störstellen entlang des Sensors reduziert und somit die Messqualität weiter verbessert werden.

Durch die Kombination einer geschirmten Elektrodengeometrie mit Innenelektrode 30 und Außenelektrode 34 sowie eines differentiellen TDR-Verfahrens kann die Fließfront eines infusionierten Harzes und/oder dessen Aushärtegrad genauer gemessen werden.

Durch die Verwendung differentieller TDR kann die Signalqualität, d.h. der Signal-zu-Rausch-Abstand, gesteigert werden. Zum Messen wird beispielsweise ein differentielles TDR-Messgerät verwendet, das über zwei Koaxialkabel mit dem Sensor verbunden wird. Die Verbindung ist derart aufgebaut, dass die Außenelektroden der Koaxialkabel kurz vor der Verbindung mit dem Sensor zusammengeführt werden. Damit ist die Verbindung zwischen Messgerät und Sensor nahezu vollständig geschirmt.

Die beschriebenen Einrichtungen, Vorrichtungen und Verfahren können insbesondere bei der Herstellung von Verbundwerkstoffen, insbesondere Faserverbundwerkstoffen, verwendet werden, wie dies auch in den eingangs erwähnten Literaturstellen näher erläutert ist.

Die Materialeigenschaften von Verbundwerkstoffen, wie beispielsweise Härte, Steifigkeit und Bruchfestigkeit, werden entscheidend bei der Herstellung bestimmt. Faserverbundwerkstoffe können allgemein aus einem Fasermaterial und einem Matrixmaterial hergestellt werden. Denkbar ist auch die Herstellung aus einem Prepreg. Als Fasermaterial werden beispielsweise Kohlenstofffasergewebe, Glasfasergewebe und dergleichen verwendet. Das Fasermaterial wird in einem sogenannten Infusionsverfahren mit dem Matrixmaterial verbunden. Dabei wird das Matrixmaterial, meist ein Kunstharz, als Bindemittel in das Fasermaterial eingespritzt. Das Matrixmaterial wird anschließend ausgehärtet. Störstellen in dem Fasermaterial oder dem Matrixmaterial, wie beispielsweise Fehlstellen oder Luftblasen, beeinträchtigen die Stabilität des Verbundwerkstoffs. Sie sind daher möglichst zu vermeiden.

Nach dem Infusionsprozess wird das Matrixmaterial in einer Konsolidierungseinrichtung ausgehärtet. Der Aushärtegrad des Matrixmaterials kann mit den hierin beschriebenen Vorrichtungen und Verfahren sowohl während des Aushärtens als auch in einer Abschlusskontrolle erfasst werden. Somit ist eine nahezu lückenlose Überwachung eines Herstellungsprozesses von Faserverbundbauteilen möglich.

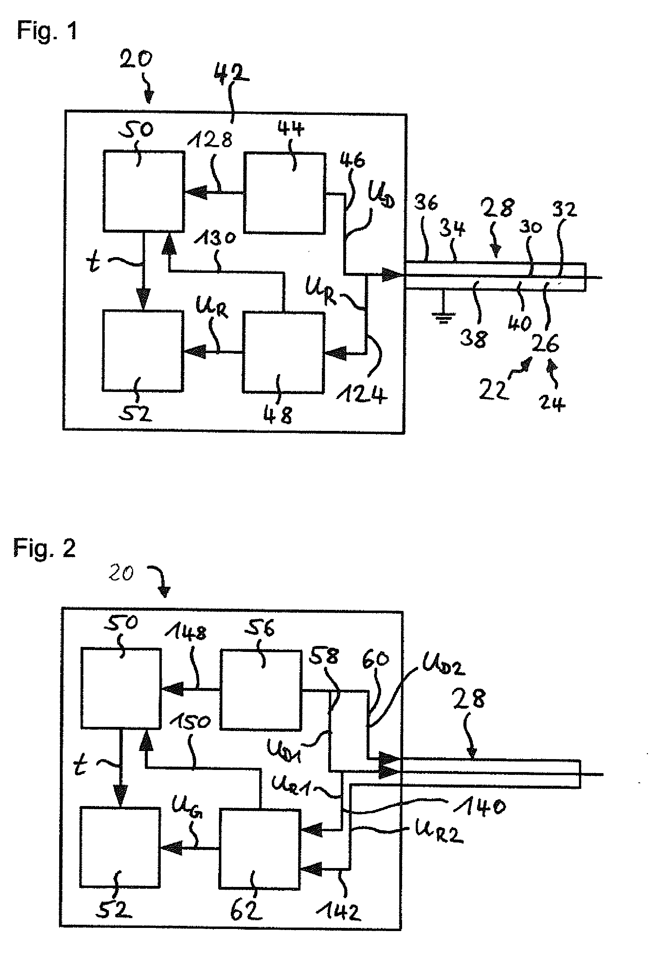

Fig. 1 zeigt schematisch ein Ausführungsbeispiel einer Materialparameterermittlungsvorrichtung 20.

Die Materialparameterermittlungsvorrichtung 20 ist ausgebildet, einen Materialparameter beispielsweise eines aus einem Fasermaterial 22 und einem Matrixmaterial 24 gebildeten Verbundwerkstoffes 26 zu ermitteln. Materialparameter sind insbesondere Fehlstellen in dem Verbundwerkstoff 26, der Aushärtungsgrad des Matrixmaterials 24 und die Verteilung des Matrixmaterials 24 in dem Verbundwerkstoff 26.

Insbesondere ist die Materialparametererfassungsvorrichtung 20 dazu ausgebildet, die Fließfront von Matrixmaterial 24 während der Herstellung von Verbundmaterialien kapazitiv zu erfassen.

Die Materialparameterermittlungsvorrichtung 20 umfasst eine Sensoreinrichtung 28. Die Sensoreinrichtung 28 erstreckt sich beispielsweise in einer axialen Richtung.

Die Sensoreinrichtung 28 weist die Innenelektrode 30 (Beispiel für eine erste Elektrode) auf.

Die Sensoreinrichtung 28 weist zudem die Außenelektrode 34 (Beispiel für eine zweite Elektrode) auf. Die Außenelektrode 34 ist aus einem elektrisch leitenden Material gebildet. Die Außenelektrode 34 umgibt im Querschnitt betrachtet die Innenelektrode 30 derart, dass sie diese bezüglich der verwendeten Felder/Frequenzen elektrisch abschirmt. Zum Beispiel umgibt die Außenelektrode 34 die Innenelektrode 30 nahezu vollständig.

Die Sensoreinrichtung 28 weist ferner wenigstens eine Probenraum 38 auf, der zwischen der Innenelektrode 30 und der Außenelektrode 34 angeordnet ist. Der Probenraum 38 wird von der Innenelektrode 30 und der Außenelektrode 34 und/oder von Isoliermaterial begrenzt, welches die Innenelektrode 30 und die Außenelektrode 34 verbindet, und ist ausgebildet, den Verbundwerkstoff 26 oder auch nur dessen Matrixmaterial 24 (z.B. während eine Infusionsprozesses) aufzunehmen. Der in dem Probenraum 38 aufgenommene Verbundwerkstoff 26 bzw. dessen Matrixmaterial 24 ist ein Beispiel für ein Dielektrikum 40.

Die Materialparameterermittlungsvorrichtung 20 umfasst zudem eine Steuereinrichtung 42. Die Steuereinrichtung 42 ist ausgebildet, die Sensoreinrichtung 28 zu steuern. Ferner ist die Steuereinrichtung 42 dazu ausgebildet, einen Materialparameter des Verbundwerkstoffs 26 zu ermitteln.

Die Steuereinrichtung 42 umfasst ein Spannungsimpulserzeugungsmodul 44. Das Spannungsimpulserzeugungsmodul 44 ist ausgebildet, einen Detektionsspannungsimpuls 46 mit einer Detektionsspannungsimpulsamplitude $U_D$ zu erzeugen. Ferner ist das Spannungsimpulserzeugungsmodul 44 dazu ausgebildet, die Sensoreinrichtung 28, insbesondere die Innenelektrode 30, mit dem Detektionsspannungsimpuls 46 zu beaufschlagen. Der Detektionsspannungsimpuls 46 kann beispielsweise eine rechteckige Impulsform aufweisen. Grundsätzlich sind auch andere Impulsformen denkbar.

Die Steuereinrichtung 42 umfasst ferner ein Spannungsimpulserfassungsmodul 48. Das Spannungsimpulserfassungsmodul 48 ist ausgebildet, eine Spannungsimpulsamplitude eines Spannungsimpulses zu erfassen.

Die Steuereinrichtung 42 umfasst weiter ein Spannungsimpulslaufzeiterfassungsmodul 50. Das Spannungsimpulslaufzeiterfassungsmodul 50 ist ausgebildet, eine Spannungsimpulslaufzeit eines Spannungsimpulses zu erfassen, wie noch beschrieben wird.

Die Steuereinrichtung 42 umfasst zudem ein Auswertemodul 52. Das Auswertemodul 52 ist ausgebildet, einen Materialparameter des Verbundwerkstoffes 26 aus einer Spannungsimpulslaufzeit und/oder einer Spannungsimpulsamplitude zu ermitteln.

Das Spannungsimpulserzeugungsmodul 44 ist mit der Innenelektrode 30 und mit dem Spannungsimpulslaufzeiterfassungsmodul 50 verbunden. Das Spannungsimpulserfassungsmodul 48 ist ebenfalls mit der Innenelektrode 30 und mit dem Spannungsimpulslaufzeiterfassungsmodul 50 verbunden. Zudem ist das Spannungsimpulserfassungsmodul 48 mit dem Auswertemodul 52 verbunden. Das Spannungsimpulslaufzeiterfassungsmodul 50 ist ebenso mit dem Auswertemodul 52 verbunden.

Es wird nachfolgend auf Fig. 2 Bezug genommen, die ein weiteres Ausführungsbeispiel der Materialparameterermittlungsvorrichtung 20 mit der Sensoreinrichtung 28 zeigt.

Die Materialparameterermittlungsvorrichtung 20 von Fig. 2 umfasst ferner ein Spannungsimpulserzeugungsmodul 56, das ausgebildet ist, einen ersten Detektionsspannungsimpuls 58 mit einer ersten Detektionsspannungsimpulsamplitude $U_{D1}$ und einen zweiten Detektionsspannungsimpuls 60 mit einer zweiten Detektionsspannungsimpulsamplitude $U_{D2}$ zu erzeugen. Das Spannungsimpulserzeugungsmodul 56 ist ferner ausgebildet, den ersten Detektionsspannungsimpuls 58 in die Innenelektrode 30 und den zweiten Detektionsspannungsimpuls 60 in die Außenelektrode 34 einzuspeisen.

Ferner umfasst die Materialparameterermittlungsvorrichtung 20 gemäß Fig. 2 ein Spannungsimpulserfassungsmodul 62, das ausgebildet ist, aus zwei eingehenden Spannungsimpulsen mit jeweils einer Spannungsimpulsamplitude, insbesondere durch Differenzbildung, eine Spannungsimpulsamplitude zu ermitteln, wie noch beschrieben wird.

Wie in der Ausführungsform von Fig. 1 umfasst die Materialparameterermittlungsvorrichtung 20 von Fig. 2 ebenfalls das Spannungsimpulslaufzeiterfassungsmodul 50 und das Auswertemodul 52.

Ähnlich wie bei dem vorigen Ausführungsbeispiel ist das Spannungsimpulserzeugungsmodul 56 mit dem Spannungsimpulslaufzeiterfassungsmodul 50 verbunden. Ferner ist das Spannungsimpulserfassungsmodul 62 mit dem Spannungsimpulslaufzeiterfassungsmodul 50 und dem Auswertemodul 52 verbunden. Wie zuvor ist das Spannungsimpulslaufzeiterfassungsmodul 50 ebenso mit dem Auswertemodul 52 verbunden.

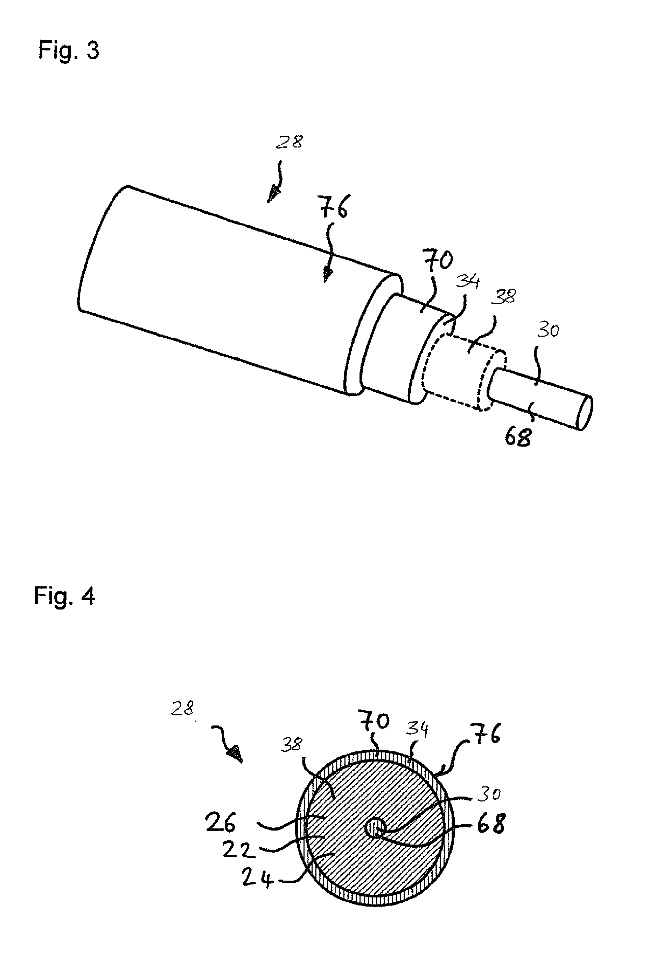

Im Folgenden wird unter Bezug auf Fig. 3 und 4 anhand eines einfachen Modells die Wirkungsweise der Sensoreinrichtung 28 näher erläutert. Die Sensoreinrichtung 28 ist hierbei stark vereinfacht wie ein Koaxialkabel dargestellt, das sich in einer axialen Richtung erstreckt. Die Innenelektrode 30 ist durch die Außenelektrode 34 abschirmend umgeben. Weiter ist der Probenraum 38 angedeutet, der z.B. zwischen der Innenelektrode 30 und der Außenelektrode 34 angeordnet ist. Die Sensoreinrichtung 30 kann eine Isolierhülle 76 aufweisen. Wie verdeutlicht wirkt die Sensoreinrichtung 30 wie ein Koaxialkabel mit Dielektrikum 40 zwischen Innenleiter und Außenleiter. Das Dielektrikum 40 wird wenigstens teilweise durch Material in dem Probenraum 38 gebildet. Ändern sich die Materialeigenschaften in dem Probenraum 38, kann dies kapazitiv und/oder mit TDR gemessen werden, und es können Orte von Defekten oder Fließfronten bestimmt werden.

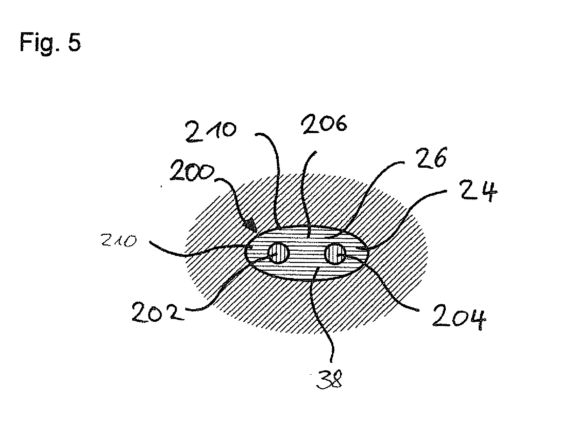

Fig. 5 zeigt sehr vereinfacht und schematisch ein Ausführungsbeispiel einer weiteren Sensoreinrichtung 200, die sich von der Sensoreinrichtung 28 dadurch unterscheidet, dass mehr als eine Innenelektrode (mehrere innere Leiter) vorgesehen sind.

Die Sensoreinrichtung 200 umfasst eine erste Innenelektrode 202 (Beispiel für eine erste Elektrode) und eine zweite Innenelektrode 204 (Beispiel für eine zweite Elektrode). Die erste Innenelektrode 202 und die zweite Innenelektrode 204 sind im Wesentlichen parallel verlaufend zueinander angeordnet und voneinander durch ein Dielektrikum 206 beabstandet. Das Dielektrikum 206 kann den Verbundwerkstoff 26 und/oder das Matrixmaterial 24 und/oder das Fasermaterial 22 aufweisen. Das Dielektrikum 206 kann auch ein Isoliermaterial 208 aufweisen, das nicht Teil des Verbundwerkstoffs 26 ist.

Ferner umfasst die Sensoreinrichtung 200 eine Außenelektrode 210, die mit Poren oder Öffnungen versehen ist und die im Querschnitt betrachtet die erste Innenelektrode 202 und die zweite Innenelektrode 204 wie oben erläutert abschirmend umgibt. Zum Beispiel umgibt die Außenelektrode 210 die Innenelektroden 202, 204 im Wesentlichen vollständig oder nahezu vollständig. Die Außenelektrode 210 begrenzt den Probenraum 38, in dem das Dielektrikum 206 oder zumindest eine Komponente des Verbundwerkstoffs 26 (z.B. dessen Matrixmaterial 24) angeordnet sein kann.

Die Außenelektrode 210 ist mit Öffnungen oder Poren derart versehen, dass Matrixmaterial 24 beim Infusionieren des Verbundwerkstoffes durch die Außenelektrode 210 hindurchtreten und in den Probenraum 38 eindringen kann.

Vorzugsweise werden bei bestimmungsgemäßer Verwendung die erste Innenelektrode 202 und die zweite Innenelektrode 204 mit Spannungsimpulsen beaufschlagt, wohingegen die Außenelektrode 210 an Erde angeschlossen ist.

Sensoreinrichtungen 28, 200 der in den Fig. 3 bis 5 vereinfacht dargestellten Art werden bevorzugt dazu eingesetzt, die Fließfront von Harz bei der CFK-Herstellung zu messen. Bisher gab es bei Sensoreinrichtungen im Stand der Technik Messfehler durch störende Einflüsse des umgebenden Mediums. Dies ist insbesondere störend bei der bevorzugten Anwendung der Fließfrontmessung bei der CFK-Herstellung, da es zu störenden Einflüssen durch CFK-Fasern und/oder durch die Formwerkzeuge oder Toolings kommt.

Als eine mögliche Lösung wird hier vorgeschlagen, die von der Fließfront abhängige Kapazität zwischen zwei Leitern (Elektroden) zu messen, wobei diese abgeschirmt sind. Vorzugsweise ist ein Kontakt zwischen Harz und den Leitern (Elektroden) möglich.

Dadurch ist eine bessere CFK-Prozesskontrolle möglich, was zu Kostenreduzierung und Qualitätsverbesserung führt.

In bevorzugter Verwendung der Sensoreinrichtungen 28, 200 wird die Fließfront gemessen, d.h. der Verlauf der Fließfront von Harz bei der Herstellung von Verbundbauteilen wie insbesondere CFK-Bauteilen.

Den Sensoreinrichtungen 28, 200 liegt folgendes Wirkprinzip zugrunde.

Harz hat eine höhere Dielektrizitätskonstante als Luft oder Vakuum. Daher kann kapazitiv gemessen werden, ob an einer Stelle Harz vorhanden ist. Eine Erhöhung der Kapazität bedeutet z.B., dass sich die Dielektrizitätskonstante erhöht hat, dass also Harz vorhanden ist.

Um das Vorhandensein von Harz nicht nur an einer Stelle zu detektieren, sondern den Verlauf der Fließfront zu erfassen, kann die Kapazität zwischen zwei parallelen Leitern gemessen werden. Wenn die Fließfront kontinuierlich fortschreitet, ist die gemessene Kapazität ein Maß für die Position der Fließfront entlang der beiden Leiter.

Die Kapazität wird allerdings durch externe Einflüsse gestört, im Wesentlichen wenn Materialien mit anderer Dielektrizitätskonstante in der Nähe sind bzw. sich der Abstand zu den beiden parallelen Leitern (d.h. zum zu messenden Kondensator) ändert. Die störenden Materialien können das CFK selber sein, aber auch Tools (z.B. metallisch) und andere Stoffe und Hilfsstoffe.

Daher werden bei den Sensoreinrichtungen 28, 200 die Leiter 30, 202, 204 gegenüber störenden Einflüssen analog zu BNC-Kabeln abgeschirmt.

Es können folgende Ansätze verfolgt werden:

a) Messung der Kapazität zwischen zwei Leitern, wobei diese von einer Abschirmung umgeben sind, oder

b) Messung der Kapazität zwischen einem Leiter und einer diesen Leiter umgebenden Abschirmung.

Die Sensoreinrichtung 28 ist für Ansatz b) geeignet, während die Sensoreinrichtung 200 für Ansatz a) geeignet ist.

Zwischen Leiter(n) (Innenelektrode(n) 30, 202, 204) und Abschirmung (Außenelektrode 34, 210) ist ein Hohlraum (Probenraum 38) ausgebildet, in den das Harz eindringen kann: Dieser Hohlraum besteht aus Luft oder einem porösen/gewebeartigen Material, in das das Harz eindringen kann.

Zur Kapazitätsmessung wird durch die Leiter oder Elektroden 30, 34; 202, 204 ein elektrisches Feld in dem Probenraum 38 erzeugt. Durch die Dielektrizität des sich im Probenraum 38 befindlichen Materials wird die Kapazität und damit das elektrische Feld beeinflusst, was man an den Leitern oder Elektroden wie beschrieben ortsauflösend erfassen kann.

Die Leiter oder Elektroden 30, 34, 202, 204 bilden somit eine Felderzeugungsanordnung 64 zur Erzeugung eines elektrischen Feldes in dem Probenraum 38, um Materialparameter des sich im Probenraum 38 befindlichen Materials, nämlich insbesondere die Dielektrizitätskonstante bzw. Permittivität, ortsauflösend zu messen.

Die Abschirmung ist perforiert, damit das Harz eindringen kann.

Der Durchmesser der Poren der Perforation ist so gewählt, dass das Harz gut eindringen kann, aber auch gute Abschirmung bei der entsprechenden Messfrequenz sichergestellt ist. Auch der Abstand zwischen Leiter(n) und Abschirmung ist entsprechend zu wählen.

Im Folgenden werden Messverfahren zur Durchführung der Messung näher erläutert.

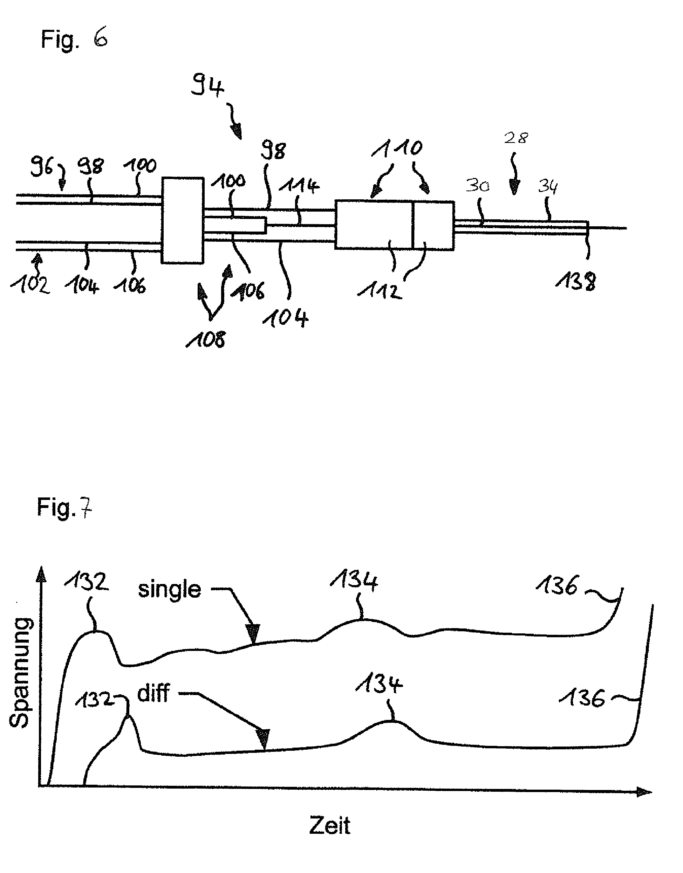

Anhand Fig. 6 wird hierzu zunächst nachfolgend eine Verbindungsanordnung 94 für die Sensoreinrichtungen 28, 200 beschrieben.

Die Verbindungsanordnung 94 umfasst ein erstes Koaxialkabel 96 mit einem ersten Innenleiter 98 und einem ersten Außenleiter 100. Ferner umfasst die Verbindungsanordnung 94 ein zweites Koaxialkabel 102 mit einem zweiten Innenleiter 104 und einem zweiten Außenleiter 106.

Die Verbindungsanordnung 94 umfasst eine erste Verbindungseinrichtung 108 zum Verbinden des ersten Koaxialkabels 96 mit dem zweiten Koaxialkabel 102. Ferner umfasst die Verbindungsanordnung 94 eine zweite Verbindungseinrichtung 110 zum Verbinden des ersten Koaxialkabels 96 und des zweiten Koaxialkabels 102 mit der Sensoreinrichtung 78. Die zweite Verbindungseinrichtung 110 weist vorzugsweise einen Steckverbinder 112 auf.

Nachfolgend wird die Verschaltung der Verbindungsanordnung 94 erläutert. Die erste Verbindungseinrichtung 108 verbindet den ersten Außenleiter 100 mit dem zweiten Außenleiter 106, so dass das erste Koaxialkabel 96 und das zweite Koaxialkabel 102 einen gemeinsamen Erdleiter 114 aufweisen. Der erste Innenleiter 98 und der zweite Innenleiter 104 werden durch den gemeinsamen Erdleiter 114 abgeschirmt. Die zweite Verbindungseinrichtung 110 wird ebenfalls durch den gemeinsamen Erdleiter 114 geschirmt. Der erste Innenleiter 98 ist beispielsweise mit der Innenelektrode 30 verbunden und der zweite Innenleiter 104 ist mit der Außenelektrode 34 verbunden. Somit kann eine Abschirmung der Zuleitung zu der Sensoreinrichtung 28, 200 bis zu dem Beginn der Sensoreinrichtung 28, 200 erreicht werden. Der Einfluss von äußeren Störsignalen ist dadurch verringert.

Es wird nachfolgend auf die Fig. 1, 7, 9A bis 9C Bezug genommen, anhand derer ein Ausführungsbeispiel eines Materialparameterermittlungsverfahrens beschrieben wird.

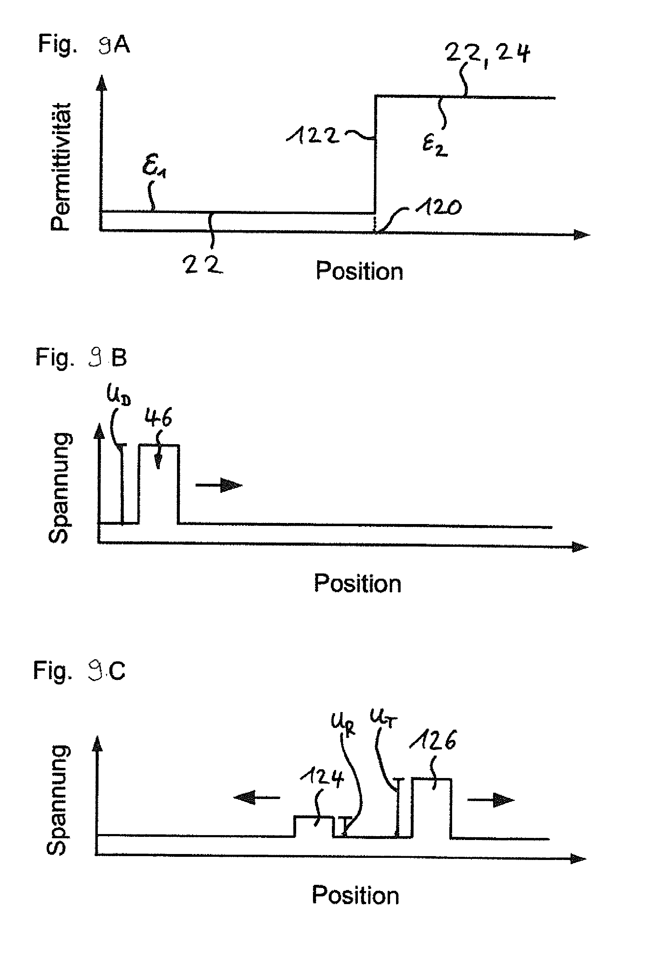

Fig. 7 zeigt schematisch ein Messergebnis einer single-ended TDR-Messung und einer differentiellen TDR-Messung. Fig. 9A zeigt schematisch den räumlichen Verlauf der dielektrischen Permittivität des Verbundwerkstoffes 26. Der Verbundwerkstoff 26 weist beispielsweise eine erste Permittivität $\varepsilon_1$ und eine zweite Permittivität $\varepsilon_2$ auf. An einer Sprungstelle 120 ist ein Permittivitätsübergang 122 zwischen der ersten Permittivität $\varepsilon_1$ und der zweiten Permittivität $\varepsilon_2$ gebildet. Der Permittivitätsübergang 122 entsteht beispielsweise durch den Übergang zwischen einem Bereich des Fasermaterials 22 ohne das Matrixmaterial 24 und einem Bereich des Fasermaterials 22 mit dem Matrixmaterial 24 oder auch Fehlern in dem Fasermaterial 22 oder in dem Matrixmaterial 24.

Fig. 9B zeigt schematisch den räumlichen Spannungsverlauf entlang der axialen Richtung einer Sensoreinrichtung, wie beispielsweise der Sensoreinrichtung 28. Schematisch dargestellt ist der Detektionsspannungsimpuls 46, der sich in Pfeilrichtung entlang der Innenelektrode 30 bewegt. Trifft der Detektionsspannungsimpuls 46 an der Sprungstelle 120 auf den Permittivitätsübergang 122, entstehen ein Reflexionsspannungsimpuls 124 mit einer Reflexionsspannungsimpulsamplitude $U_R$ und ein Transmissionsspannungsimpuls 126 mit einer Transmissionsspannungsimpulsamplitude $U_T$. Es gilt im Wesentlichen:

$$U_R \ \propto \ \frac{\sqrt{\varepsilon_1} - \sqrt{\varepsilon_2}}{\sqrt{\varepsilon_1} + \sqrt{\varepsilon_2}}$$

Der Reflexionsspannungsimpuls 124 läuft von der Sprungstelle 120 zurück zu der Materialparameterermittlungsvorrichtung 20 und wird dort von dem Spannungsimpulserfassungsmodul 48 erfasst. Insbesondere wird die Reflexionsspannungsimpulsamplitude $U_R$ von dem Spannungsimpulserfassungsmodul 48 erfasst. Beim Aussenden des Detektionsspannungsimpulses 46 wird von dem Spannungsimpulserzeugungsmodul 44 ein Startsignal 128 an das Spannungsimpulslaufzeiterfassungsmodul 50 gesendet. Beim Empfangen des Reflexionsspannungsimpulses 124 sendet das Spannungsimpulserfassungsmodul 48 ein Stoppsignal 130 an das Spannungsimpulslaufzeiterfassungsmodul 50. Das Spannungsimpulslaufzeiterfassungsmodul 50 ermittelt den Zeitabstand zwischen dem Startsignal 128 und dem Stoppsignal 130 und daraus eine Spannungsimpulslaufzeit t. Die Spannungsimpulslaufzeit t und die Reflexionsspannungsimpulsamplitude $U_R$ werden an das Auswertemodul 52 übermittelt. Das Auswertemodul 52 bestimmt aus der Spannungsimpulslaufzeit t und dem Verhältnis aus Reflexionsspannungsimpulsamplitude $U_R$ und Detektionsspannungsimpulsamplitude $U_D$ das Verhältnis von erster Permittivität $\varepsilon_1$ und zweiter Permittivität $\varepsilon_2$ sowie die Position der Sprungstelle 120 in der Zeitdomäne.

Da die dielektrischen Eigenschaften des Fasermaterials 22 und des Matrixmaterials 24 gut bekannt sind, bestimmt das Auswertemodul 52 daraus die räumliche Position der Sprungstelle 120 entlang der Sensoreinrichtung 78 und die Höhe des Permittivitätsübergangs 122, d.h. die Änderung zwischen erster Permittivität $\varepsilon_1$ und zweiter Permittivität $\varepsilon_2$.

Als Ergebnis erhält man beispielsweise die in der Fig. 7 mit "single" bezeichnete Messkurve. Es sind im Wesentlichen eine erste Sprungstelle 132, eine zweite Sprungstelle 134 und eine dritte Sprungstelle 136 zu erkennen. Die erste Sprungstelle 132 kann auf den Anschluss der Sensoreinrichtung 28 zurückgeführt werden. Die zweite Sprungstelle 134 gibt beispielsweise die momentane Position der Fließfront des Matrixmaterials 22 an. Aus dem Amplitudenverhältnis des Reflexionsspannungsimpulsamplitude $U_R$ und der Detektionsspannungsimpulsamplitude $U_D$ lässt sich der Aushärtungsgrad des Matrixmaterials 24 ableiten. Die dritte Sprungstelle 136 ist stark ausgeprägt und kann auf einen Endabschnitt 138 der Sensoreinrichtung 28 zurückgeführt werden. Der Endabschnitt 138 weist aufgrund des abrupten Übergangs zwischen der Sensoreinrichtung 28 und Luft eine hohe Reflektivität auf, so dass die Reflexionsspannungsimpulsamplitude $U_R$ entsprechend groß ist.

Es wird nachfolgend auf die Fig. 2, 7, 8, 10A bis 10C Bezug genommen, anhand derer ein Ausführungsbeispiel eines Materialparameterermittlungsverfahrens beschrieben wird.

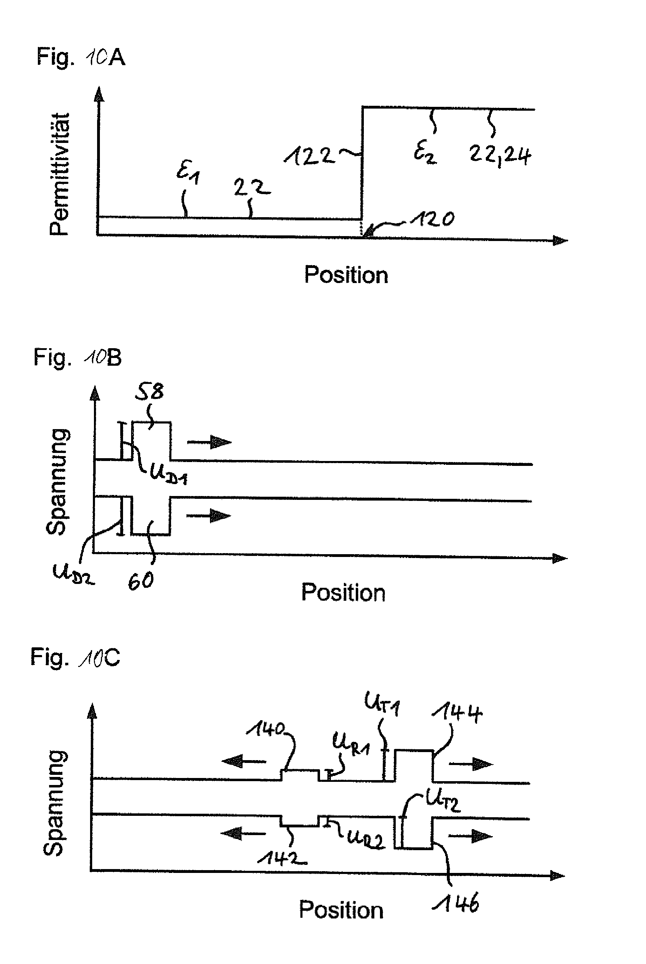

Fig. 10A zeigt schematisch die gleiche Darstellung wie Fig. 9A, so dass diese nicht näher beschrieben wird.

Fig. 10B zeigt schematisch den jeweils von der Materialparameterermittlungsvorrichtung 20 erzeugten ersten Detektionsspannungsimpuls 58 und den zweiten Detektionsspannungsimpuls 60. Wie aus Fig. 10B ersichtlich, haben der erste Detektionsspannungsimpuls 58 und der zweite Detektionsspannungsimpuls eine gegengleiche Spannungsimpulsamplitude. Es gilt:

$$U_{D1} = -U_{D2}$$

Der erste Detektionsspannungsimpuls 58 und der zweite Detektionsspannungsimpuls 60 bewegen sich entlang der axialen Richtung der Sensoreinrichtung 28 jeweils in der Innenelektrode 30 und der Außenelektrode 34.

Treffen der erste Detektionsspannungsimpuls 58 und der zweite Detektionsspannungsimpuls 60 auf den Permittivitätsübergang 122 an der Sprungstelle 120, so entstehen jeweils ein erster Reflexionsspannungsimpuls 140 mit einer ersten Reflexionsspannungsimpulsamplitude $U_{R1}$ und ein zweiter Reflexionsspannungsimpuls 142 mit einer zweiten Reflexionsspannungsimpulsamplitude $U_{R2}$. Ebenso entstehen jeweils ein erster Transmissionsspannungsimpuls 144 mit einer ersten Transmissionsspannungsimpulsamplitude $U_{T1}$ und ein zweiter Transmissionsspannungsimpuls 146 mit einer zweiten Transmissionsspannungsimpulsamplitude $U_{T2}$. Es gilt im Wesentlichen:

$$U_{Ri} \propto \frac{\sqrt{\varepsilon_1} - \sqrt{\varepsilon_2}}{\sqrt{\varepsilon_1} + \sqrt{\varepsilon_2}}; i = 1, 2$$

Beim Aussenden des ersten Detektionsspannungsimpulses 58 und des zweiten Detektionsspannungsimpulses 60 sendet das Spannungsimpulserzeugungsmodul 56 ein Startsignal 148 an das Spannungsimpulslaufzeiterfassungsmodul 50. Beim Empfangen des ersten Reflexionsspannungsimpulses 140 und des zweiten Reflexionsspannungsimpulses 142 sendet das Spannungsimpulserfassungsmodul 62 ein Stoppsignal 150 an das Spannungsimpulslaufzeiterfassungsmodul 50. Ferner ermittelt das Spannungsimpulserfassungsmodul 62 eine Gesamtreflexionsspannungsimpulsamplitude $U_G$ aus der ersten Reflexionsspannungsimpulsamplitude $U_{R1}$ und der zweiten Reflexionsspannungsimpulsamplitude $U_{R2}$. Es gilt:

$$U_G = U_{R1} - U_{R2}$$

Die Gesamtreflexionsspannungsimpulsamplitude $U_G$ wird an das Auswertemodul 52 geleitet und ebenso wie die Reflexionsspannungsimpulsamplitude $U_R$ des vorigen Beispiels verarbeitet.

In Fig. 7 ist das Messergebnis dieser sogenannten differentiellen TDR-Messung mit "diff" bezeichnet. Durch die Nutzung differentieller TDR zur Überwachung der Fließfront und des Aushärtegrades kann der Einfluss leitender Materialien in der Umgebung der Sensoreinrichtung 28 weiter reduziert und die Genauigkeit solcher Messungen in derartigen Umgebungen gegenüber der single-ended Messung weiter verbessert werden. Dies ist unter anderem darauf zurückzuführen, dass der erste Detektionsspannungsimpuls 58 und der zweite Detektionsspannungsimpuls 60 von äußeren Einflüssen gleichermaßen betroffen sind, so dass diese bei der Differenzbildung eliminiert werden.

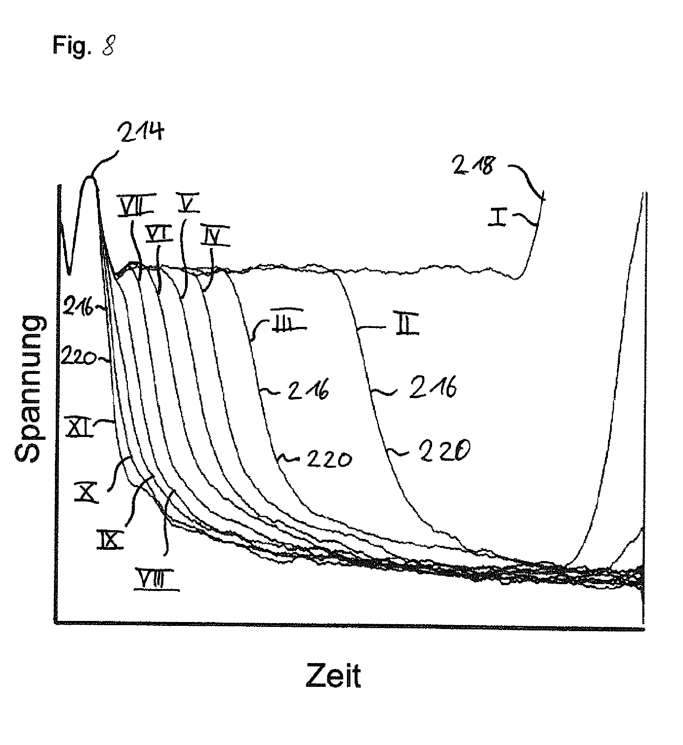

In Fig. 8 ist beispielhaft ein Messergebnis einer differentiellen TDR-Messung dargestellt, wie es beispielsweise durch die Messung mit der Sensoreinrichtung 200 mit mehreren Innenelektroden erhalten wird. In Fig. 8 ist die erfasste Spannung gegenüber der Zeit aufgetragen.

Die Messkurven werden mit I bis XI bezeichnet. Die Messkurve I ist im "trockenen" Zustand der Sensoreinrichtung 200 aufgenommen. In diesem Zustand befindet sich lediglich das Dielektrikum 206 in dem Probenraum 212. Das Fasermaterial 22 ist außerhalb der Außenelektrode 210 vorgesehen.

In Messkurve I sind eine erste Sprungstelle 214 und eine dritte Sprungstelle 218 erkennbar. Die erste Sprungstelle 214 rührt von dem Übergang in die Sensoreinrichtung 200 her. Die dritte Sprungstelle 218 ist auf einen Endabschnitt der Sensoreinrichtung 200 zurückzuführen.

Sodann wird die Infusion des Fasermaterials 22 begonnen. Das Matrixmaterial 24 breitet sich in dem Fasermaterial 22 aus. Trifft das Matrixmaterial 24 auf die Sensoreinrichtung 200, dringt das Matrixmaterial 24 durch die mit Poren oder Öffnungen versehene Außenelektrode 210 in den Probenraum 38 ein. Eine Fließfront des Matrixmaterials 24 erzeugt einen Permittivitätsübergang 220 bei einer zweiten Sprungstelle 220. Es entsteht eine Messkurve ähnlich der Kurve II.

Wie aus der Messkurve II ersichtlich ist befindet sich der Permittivitätsübergang 220, d. h. die Fließfront des Matrixmaterials 24, etwa auf halber Länge der Sensoreinrichtung 200. Mit zunehmender Infusionszeit dringt mehr Matrixmaterial 24 in die Sensoreinrichtung 200 ein. Die Fließfront des Matrixmaterials 24 wandert immer weiter entlang der Sensoreinrichtung 200. Der Permittivitätsübergang 220 wandert somit weiter in Richtung der ersten Sprungstelle 214 (Messkurve III bis X). Schließlich wird ein stabiler Endzustand erreicht, wie er durch die Messkurve XI dargestellt ist. In diesem Zustand hat das Matrixmaterial 24 die Sensoreinrichtung 200 nahezu vollständig ausgefüllt.

Die Materialparameterermittlungsvorrichtungen 20, 54 können in einer Variante auch auf die Stoppsignale 130, 150 verzichten. Stattdessen wird dann lediglich das Startsignal 128, 148 an das Spannungsimpulslaufzeiterfassungsmodul 50 gesendet. Ferner wird kontinuierlich die momentan an dem Spannungsimpulserfassungsmodul 48, 62 anliegende Spannung an das Spannungsimpulslaufzeiterfassungsmodul 50 übermittelt und mit einem Zeitstempel versehen. Der Zeitstempel umfasst die seit dem erhalten des Startsignales 128, 148 verstrichene Zeit. Die Wertepaare Zeitstempel und Spannung werden an das Auswertemodul 52 zum Auswerten übermittelt. Es ist auch denkbar, dass die seit dem Erhalten des Startsignales 128, 148 verstrichene Zeit unabhängig von der momentan anliegenden Spannung an das Auswertemodul 52 übertragen wird.

Mit den beschriebenen Vorrichtungen und Verfahren lässt sich der Herstellungsprozess des Verbundwerkstoffes 26 überwachen. Insbesondere können Fehler beim Infusionieren und Aushärten des Matrixmaterials 24 erfasst werden. Ferner kann beim Aushärten des Matrixmaterials 24 der Aushärtegrad ermittelt werden. Ebenso kann die Position oder die Verteilung der Fließfront 220 des Matrixmaterials 24 in dem Fasermaterial 22 oder dem Verbundwerkstoff 26 bestimmt werden. Damit lässt sich der Herstellungsprozess des Verbundwerkstoffes 26 umfangreicher und genauer als bisher überwachen und steuern. Die Herstellung des Verbundwerkstoffes kann so weiter automatisiert und somit weniger aufwändig gestaltet werden. Verbleiben die Sensoreinrichtungen 28, 200 nach dem Aushärten in dem Verbundwerkstoff 26, kann eine Überwachung des Verbundwerkstoffes 26 auch im verbauten Zustand durchgeführt werden. Beschädigungen bei der Nutzung und Alterungsprozesse können somit ebenfalls nachvollzogen werden.

Im Folgenden werden der konkrete Aufbau von bevorzugten Ausgestaltungen der Sensoreinrichtungen 28, 200 sowie mögliche Herstellungsverfahren für die Sensoreinrichtungen näher erläutert.

Die Sensoreinrichtungen 28, 200 werden vorzugsweise mit PCB-Technologie oder Leiterplattentechnologie realisiert.

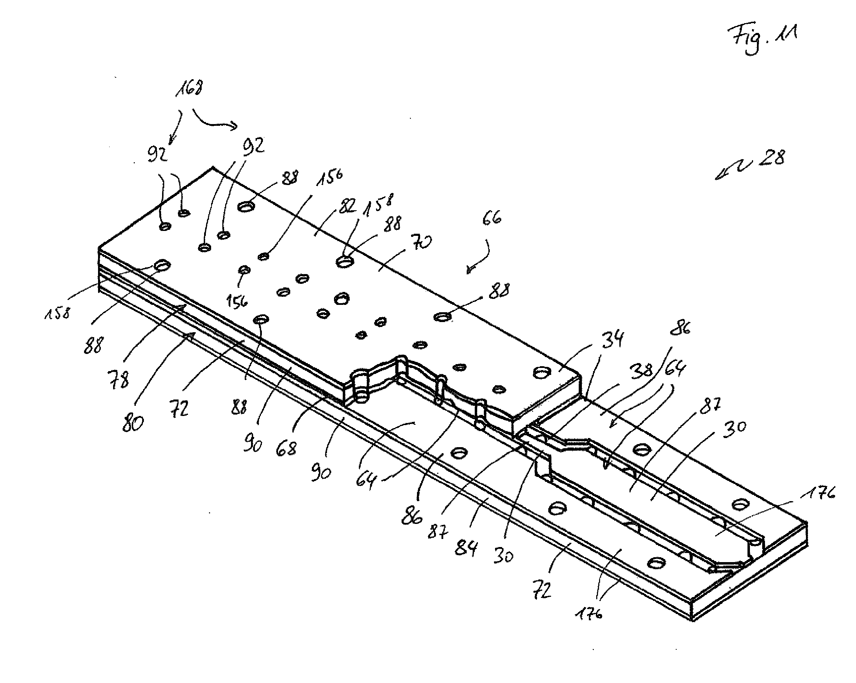

Die Fig. 11 bis 13 zeigen eine perspektivische Darstellung, eine Draufsicht und eine Schnittdarstellung einer bevorzugten Ausgestaltung der Sensoreinrichtung 28.

Die Sensoreinrichtung 28 weist eine Schichtstruktur 66 aus elektrisch leitenden Schichten 68, 70 und elektrisch isolierenden Schichten 72 auf. An oder innerhalb der Schichtstruktur 66 ist der wenigstens eine Probenraum 38 gebildet.

Die Sensoreinrichtung 28 ist insgesamt als längliches Plattenelement gebildet. Elektrisch leitende Schichten 68, 70 bilden die Leiter oder Elektroden 30, 34. Durch die Elektroden 30, 34 ist die Felderzeugungsanordnung 64 gebildet, mittels der sich wie oben beschrieben die Permittivität von sich im Probenraum 38 befindlichen Material ortsauflösend entlang der Längsrichtung des länglichen Plattenelements messen lässt.

Dabei bildet eine wenigstens eine innere erste elektrische Schicht 68 die Innenelektrode 38, während äußere zweite elektrische Schichten, die elektrisch verbunden sind, als Abschirmung und Außenelektrode 34 wirken.

In dem in den Fig. 11 bis 13 gezeigten Ausführungsbeispiel ist die Schichtstruktur 66 aus zwei Schichtstrukturmodulen 82, 84 aufgebaut, die beispielsweise durch zwei Leiterplatten 78, 80 gebildet sind. Die Leiterplatten 78, 80 sind beidseitig mit einer elektrisch leitenden Schicht 68, 70 versehen. Die zueinander weisenden inneren elektrisch leitenden Schichten sind derart strukturiert, dass äußere Randbereiche 86 und ein innen verlaufender Innnenbereich 87 elektrisch getrennt voneinander sind.

Die Innenbereiche 87 der elektrisch leitenden inneren Schicht 68 bilden die Innenelektrode 30. Die Randbereiche 86 sind durch Durchkontaktierungen 88 mit den äußeren elektrisch leitenden Schichten 70 elektrisch leitend verbunden. Dadurch bilden die äußeren elektrisch leitenden Schichten 70 und die Randbereiche 86 die Außenelektrode 34, die die Innenelektrode 30 abschirmend umgibt. Die durch die Leiterplattensubstrate 94 gebildeten elektrisch isolierenden Schichten 72 bilden ein Gehäuse, innerhalb dem der längliche Probenraum 38 entlang der Innenelektrode 30 ausgebildet ist. Entlang des Probenraums 38 sind eine Reihe von Öffnungen in Form von Infiltrationsbohrungen durch die äußeren elektrisch leitenden Schichten 70 und die elektrisch isolierenden Schichten 72 bis in den Probenraum 38 hinein geführt. Diese Öffnungen 70 sind als Infiltrationsbohrungen 92 ausgeführt.

Bei Einsatz der Sensoreinrichtung 28 zur Überwachung eines CFK-Herstellprozesses kann je nach Fortschreiten der sich in Längsrichtung der Sensoreinrichtung 28 bewegenden Fließfront Harz oder dergleichen Matrixmaterial 24 in mehr oder weniger Infiltrationsbohrungen 92 eintreten und so entsprechend der Fortbewegung der Fließfront den Probenraum 38 auf entsprechender Länge füllen.

Ein erstes Schichtstrukturmodul 82 ist an einem Anschlussende der Sensoreinrichtung 28 kürzer ausgeführt als das zweite Schichtstrukturmodul, so dass an der Innenseite des zweiten Schichtstrukturmoduls der den Innenleiter bildende Innenbereich 87 - Innenelektrode 30 - und die äußeren Randbereiche 86 - Teil des Außenleiters bzw. der Außenelektrode 34 - frei liegen. Der frei liegende Bereich des Innenbereichs 87 ist gegenüber dem in dem Probenraum 38 verlaufenden Bereich mit größerer Breite ausgeführt. Dadurch sind Anschlussflächen 152 zum Anschließen der Innenelektrode 30 und Außenelektrode 34 geschaffen.

Die elektrisch leitenden Schichten 68, 70 sind beispielsweise aus verzinntem Kupfer ausgeführt, während die elektrisch isolierende Schicht 72 aus FR4 ausgeführt ist.

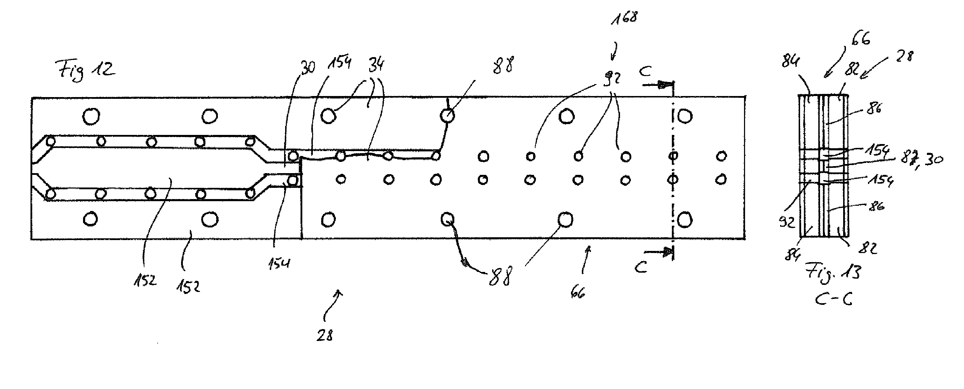

Wie aus Fig. 13 ersichtlich, weist der Probenraum 38 mehrere beidseitig der Innenelektrode 30 ausgebildete Infiltrationskanäle 154 auf, in die die Infiltrationsbohrungen 92 münden.

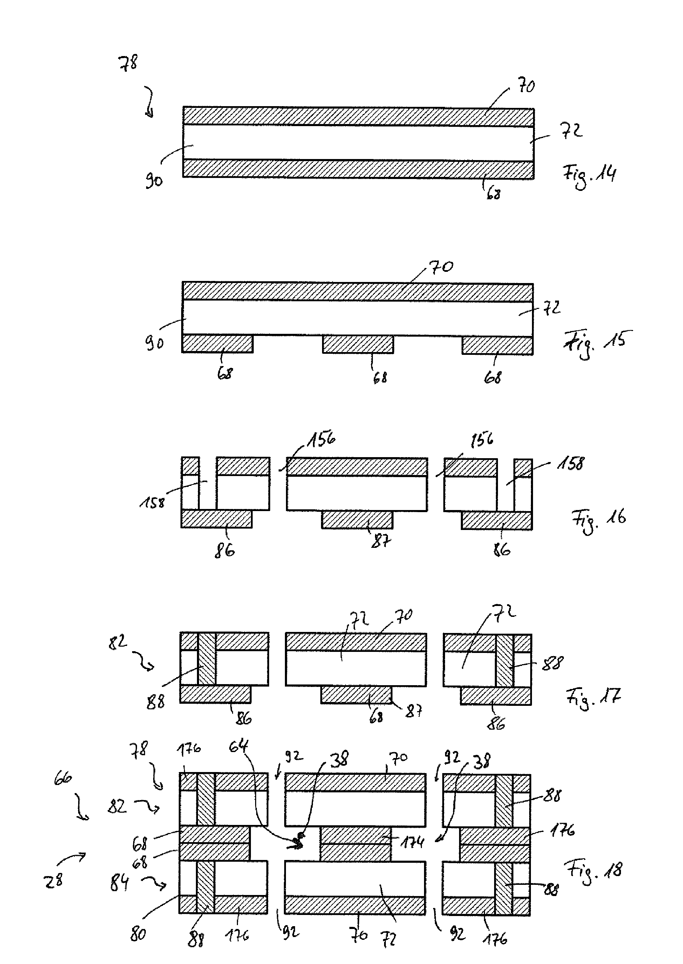

Im Folgenden wird anhand der Darstellungen in den Fig. 14 bis 18 ein Herstellverfahren zum Herstellen der in den Fig. 11 bis 13 gezeigten Ausführungsform der Sensoreinrichtung 28 näher erläutert.

Wie in Fig. 14 dargestellt, geht man von einer ersten Leiterplatte 78 aus, die beidseitig mit elektrisch leitenden Schichten 68, 70, wie z.B. aus Kupfer, beschichtet ist.

Wie in Fig. 15 dargestellt, wird die erste elektrisch leitende Schichten 68 einseitig strukturiert, um Leiterbahn zu realisieren.

Gemäß Fig. 16 fertigt man anschließend erste Bohrungen 156, um die untere Kupferbahn - erste elektrisch leitende Schicht 68 - kontaktieren zu können, und zweite Bohrungen 158, durch die später Harz eindringen kann.

Anschließend fertigt man gemäß Fig. 17 Kontaktierung (Via) zu der unteren Kupferbahn, um so die Durchkontaktierungen 88 zu bilden. Dadurch erhält man das erste Schichtstrukturmodul 82.

Anschließend oder parallel wird in analoger Weise das identisch zu dem ersten Schichtstrukturmodul 82 ausgebildete zweite Schichtstrukturmodul 84 hergestellt.

Gemäß Fig. 18 werden zwei solcher Schichtstrukturmodule 82, 84 spiegelbildlich zueinander verbunden, um so die Schichtstruktur 66 der Sensoreinrichtung 28 zu erhalten.

Das Verbinden erfolgt z.B., indem Kupfer verzinnt wird. Es können zwei identische Strukturmodule übereinander gelegt (face-to-face) werden. Diese können verlötet werden.

In nicht näher dargestellten Alternativen können die Infiltrationsbohrungen 92 nur auf einer Seite oder aber auf beiden Seiten hergestellt werden.

Die bisherigen Ausführungsformen nutzen PCB-Technologien und Materialabtrag von beidseitigen Leiterplatten zur Herstellung. Dagegen nutzen im Folgenden näher beschriebene Verfahren weitaus dünnere Substrate und Materialauftragstechniken, um dünnere Schichtstrukturen 66 zu erzeugen.

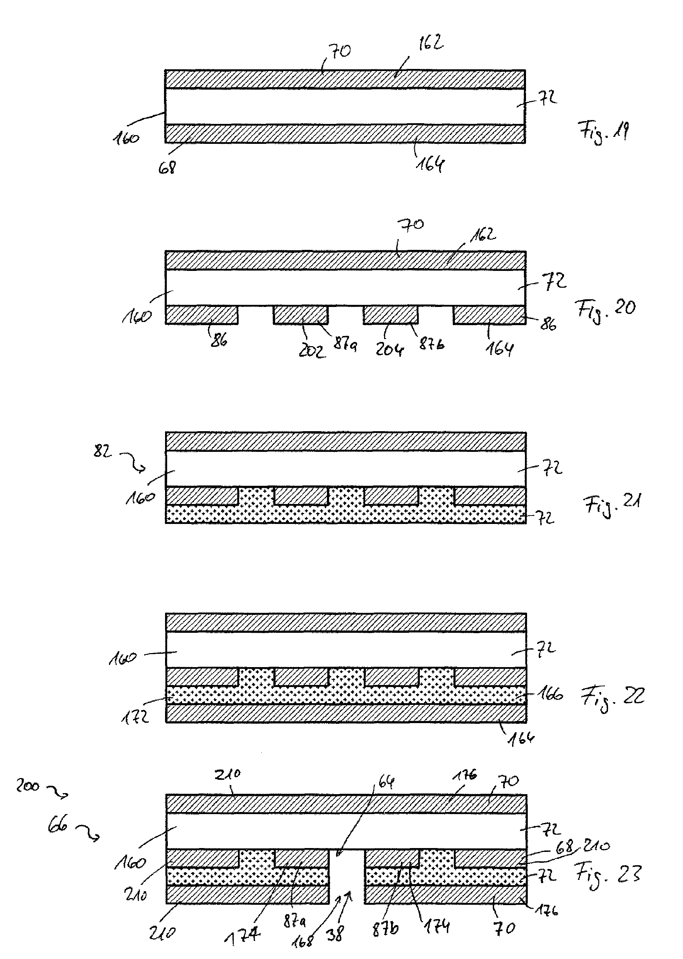

Bei den Ausführungsformen der Fig. 19 bis 35 wird demnach als Substrat eine dünne Folie 160 verwendet, die die wenigstens eine elektrisch isolierende Schicht 72 bildet. Die Folie 160 kann z.B. aus Kapton, PEI, Polyamid oder einem anderen geeigneten elektrisch isolierenden Folienmaterial gebildet sein.

Im Folgenden werden anhand der Darstellungen der Fig. 19 bis 23 ein mögliches Herstellverfahren und dessen Varianten erläutert.

Wie in Fig. 19 dargestellt, wird die Folie 160 auf der einen Seite (Oberseite) mit einer ganzflächigen Metallisierung 162 versehen.

Auch die andere Seite (Unterseite) kann, wie in Fig. 19 dargestellt, mit einer ganzflächigen Metallisierung 164 versehen werden.

Das Metallisieren kann z.B. durch Auflaminieren, Siebdruck, Spray und/oder physikalische Gasphasenabscheiden, z.B. Sputtern erfolgen.

Die Metallisierung 162 auf der anderen Seite kann zunächst ganzflächig erfolgen, wobei daraufhin die so gebildete elektrisch leitfähige Schicht 70 strukturiert wird. Dies ergibt dann den in Fig. 20 dargestellten Schichtstrukturrohling.

Der strukturierte elektrische leitfähige Schicht 70 des Schichtstrukturrohlings von Fig. 20 kann aber alternativ auch durch selektive Metallisierung hergestellt werden, z.B. mittels Inkjet- oder Aerosoljet-Druck, mittels Siebdruck und/oder Lift-off-Technik.

Anschließend erfolgt ein Aufbringen eines Isolators 166, z.B. aus Fotolack oder besserem temperaturstabilem Material z.B. durch Spin-Coating, Spray, Siebdruck oder physikalische Gasphasenabscheidung (PVD) oder ein vergleichbares Verfahren. Dies ergibt die in Fig. 21 gezeigte Struktur.

Gemäß Fig. 22 wird anschließend Metall auf der Unterseite, d.h. auf dem Isolator 166 aufgebracht. Dadurch ist der Schichtstrukturrohling mit zwei äußeren elektrisch leitenden Schichten 70 und einer strukturierten inneren elektrisch leitenden Schicht 68 versehen, deren Innenbereich in einen Innenbereich 87 und zwei Randbereiche 86 aufgeteilt werden kann, um die Sensoreinrichtung 28 zu bilden, oder in zwei voneinander getrennte Innenbereiche 87a und 87b und zwei Randbereiche 86 aufgeteilt sein kann, um die Sensoreinrichtung 200 mit den beiden Innenelektroden 202, 204 zu bilden.

Anschließend erfolgt eine elektrische Kontaktierung der leitenden Bereiche z.B. mittels Durchkontaktierungen 88.

Danach erfolgt das Einbringen einer Infiltrationsöffnung in die Folienstruktur, z.B. durch Laser oder photolithografisch/Ätzen, von beliebiger Seite durch den ganzen Aufbau hindurch bis zur Tiefe, in der die Leiter liegen. In einem Beispiel ist der Isolator 166 aus Si3N4 oder SiO2 über PVD-Verfahren gebildet. Hierbei können das Metall der äußeren elektrisch leitenden Schicht 70 und der Isolator 166 konventionell photolithografisch strukturiert und geätzt werden. Das Ergebnis dieses Schritts ist in Fig. 23 dargestellt.

Im Gegensatz zu den bisherigen Beispielen sind einfachen Bohrungen bzw. Sacklöcher weniger geeignet, es sollte stattdessen eine Nut über die gesamte Länge hinweg entstehen, um die Infiltrationsöffnung 168 zu bilden.

Auch alternative Schrittabfolgen sind möglich.

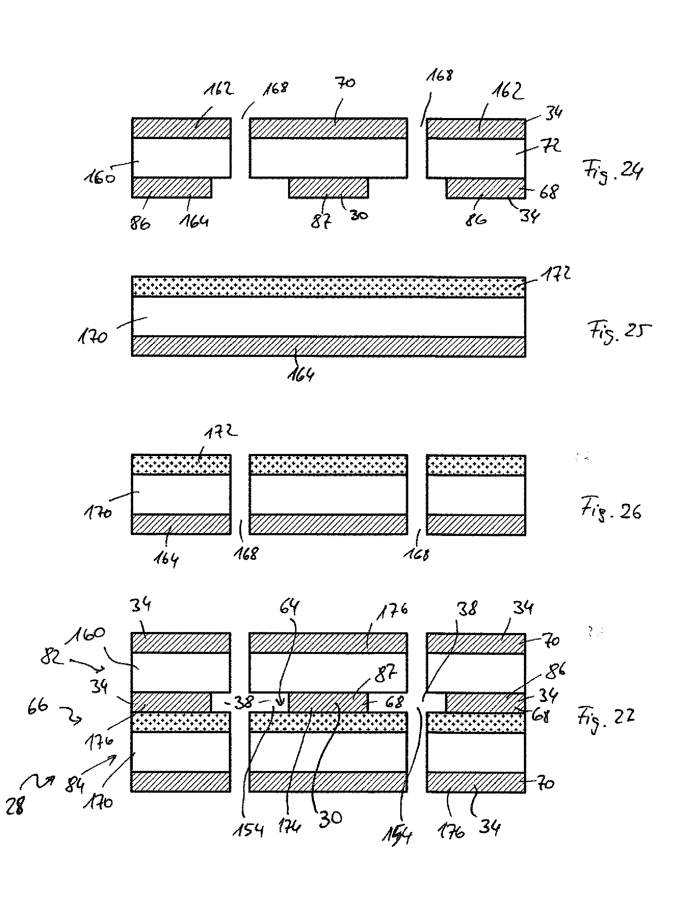

Im Folgenden wird ein alternatives Herstellverfahren anhand der Fig. 24 bis 27 erläutert.

Auch hierbei wird eine dünne Folie 160 (Kapton oder PEI oder Polyamid oder andere) für die Oberseite verwendet.

Anschließend erfolgt eine ganzflächige Metallisierung auf beiden Seiten, z.B. durch durch Auflaminieren, Siebdruck, Spray oder physikalische Gasphasenabscheidung z.B Sputtern mit anschließender Strukturierung und/oder selektive Metallisierung mittels Inkjet- bzw. Aerosoljet-Druck oder Siebdruck.

Anschließend erfolgt das Einbringen einer oder mehrerer Infaltrationsöffnungen 168, z.B. Infiltrationsbohrung 92 in die Folie 160, z.B. durch Laser oder dergleichen.

Das Ergebnis ist in Fig. 24 dargestellt. Fig. 24 zeigt das erste Schichtstrukturmodul 82 der späteren Schichtstruktur 66 der Sensoreinrichtung 28.

Weiter wird eine weitere dünne Folie 170 (z.B. Kapton oder PEI oder Polyamid oder andere) für die Unterseite verwendet.

Es folgt ganzflächiges Metallisierung auf einer der Seiten der weiteren Folie 170, z.B. durch durch Auflaminieren, Siebdruck, Spray oder physikalische Gasphasenabscheidung z.B Sputtern.

Weiter erfolgt Aufbringen einer Verbindungsschicht 172 (z.B. Kleber). Hierzu kann z.B. ein geeignetes flüssiges Monomer oder Polymer z.B. durch Spin-Coating, Spray oder Siebdruck oder ein vergleichbares Verfahren aufgebracht werden. Das Ergebnis ist in Fig. 25 dargestellt.

Anschließend wird auch hier wenigstens eine Infiltrationsöffnung 168, z.B. in Form von mehreren Infiltrationsbohrungen 92 in die weitere Folie 170, z.B. durch Laser eingebracht.

Das sich so ergebende zweite Schichtstrukturmodul 84 ist in Fig. 26 dargestellt.

Die beiden durch die Folien 160, 170 gebildeten Schichtstrukturmodule 82, 84 werden anschließend aufeinander laminiert. Dies ergibt die in Fig. 27 dargestellte Schichtstruktur 66.

Abweichend zu der zuvor erläuterten Verfahrensweise können eine oder beider der Folien 160, 170 auch erst zum Schluss perforiert werden.

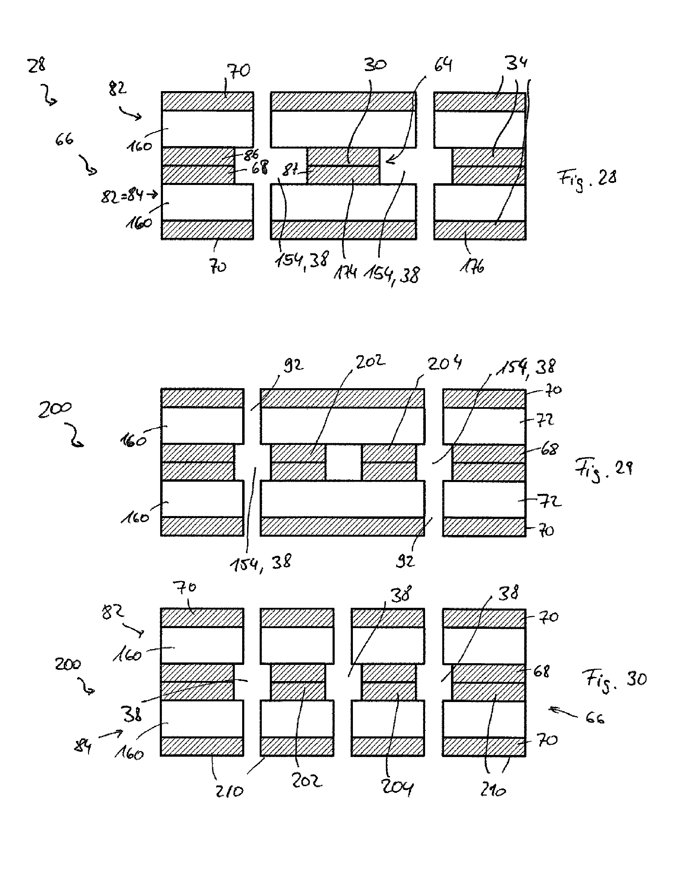

Gemäß einer alternativen Verfahrensweise, die in Fig. 28 dargestellt ist, können analog zum PCB-Ansatz auch zwei Folien 160 zusammengefügt werden, z.B. durch Kleben oder Niedertemperatur-Löten. Hier sind dann zwei erste Schichtstrukturmodule 82, die wie oben anhand der Fig. 24 erläutert hergestellt sind, spiegelbildlich zusammengefügt.

Die Folie 160, 170 ist nicht starr. Während Harz nach innen fließt, könnte ein Druckunterschied zwischen innen und außen auftreten. Dadurch könnte die Folie 160, 170 komprimiert werden. Hierdurch könnte sich eine Änderung des Abstands zwischen Leiter und Abschirmung ergeben. Dies kann zu einer Kapazitätsänderung und somit zu einem Messfehler führen.

Eine mögliche konstruktive Abhilfe wird im Folgenden anhand der Darstellung in Fig. 29 erläutert.

Bei der in Fig. 29 dargestellten, ähnlich wie bei der Fig. 28 hergestellten Schichtstruktur 66 erfolgt eine Messung der Kapazität zwischen zwei parallelen Leitern - erste und zweite Innenelektrode 202, 204 -, die von einer Abschirmung - Außenelektrode 210 - umgeben sind.

Mit dieser Anordnung kann bei der Electrical Time Domain Reflectometry (E-TDR) auch differentiell gemessen werden.

Eine Alternative ist in Fig. 30 dargestellt; hier ist ein Teil des Probenraums 38 auch im Inneren zwischen den beiden Innenelektroden 202, 204 ausgebildet.

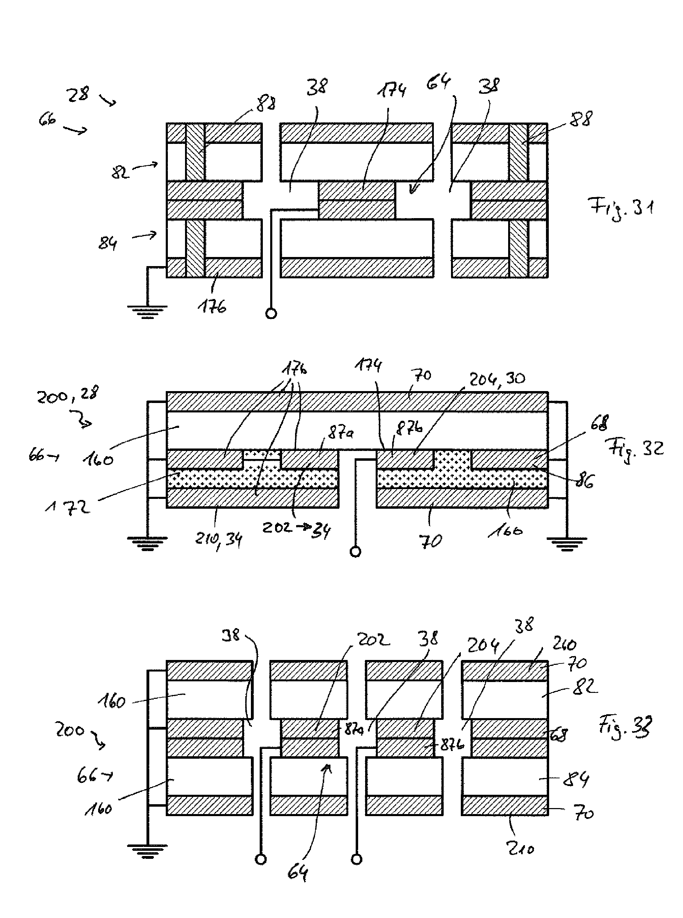

Im Folgenden werden anhand der Darstellungen der Fig. 31 bis 33 noch mögliche elektrische Anschlüsse der Sensoren erläutert.

Bei der Darstellung von Fig. 1 ist nur ein Innenleiter mit Abschirmung vorgesehen. Der Innenleiter ist durch eine elektrisch leitfähige Messschicht 174 der Schichtstruktur 66 ausgebildet. Die Abschirmung ist durch Erdungsschichten 176 der Schichtstruktur 66 gebildet.

In Fig. 32 ist eine Zwei-Leiter-Anordnung mit zwei Innenleitern dargestellt, davon ist ein Leiter mit der Abschirmung verbunden (dies entspricht dann im Wesentlichen der Ein-Leiter-Anordnung).

In Fig. 33 ist eine Zwei-Leiter-Anordnung dargestellt, wobei beide Leiter separat geführt werden und so eine differenzielle Messung ermöglichen. Dies entspricht dann der Sensoranordnung 200 mit zwei Innenelektroden 202, 204 und einer abschirmenden Außenelektrode 210 auf Masse.

Die zuvor beschriebenen Vorrichtungen und Verfahren ermöglichen die Messung der Fließfront, d.h. des Verlaufs der Fließfront von Harz bei der Herstellung von Verbundbauteilen, wie etwa CFK-Bauteilen.

Harz hat im Allgemeinen eine höhere Dielektrizitätskonstante als Luft oder Vakuum. Es kann somit kapazitiv gemessen werden, ob an einer Stelle Harz vorhanden ist. Eine Erhöhung der Kapazität bedeutet dabei, dass sich die Dielektrizitätskonstante erhöht hat, dass also Harz vorhanden ist. Um das Vorhandensein von Harz nicht nur an einer Stelle zu detektieren, sondern den Verlauf der Fließfront zu erfassen, kann die Kapazität zwischen zwei parallelen Leitern gemessen werden. Wenn die Fließfront kontinuierlich fortschreitet, ist die gemessene Kapazität ein Maß für die Position der Fließfront entlang der beiden Leiter.

Dabei tritt jedoch allgemein das Problem auf, dass die Kapazität durch externe Einflüsse gestört wird. Dies tritt im Wesentlichen auf, wenn Materialien mit anderer Dielektrizitätskonstante in der Nähe sind und/oder sich der Abstand zu den beiden parallelen Leitern (d.h. zum zu messenden Kondensator) ändert. Die störenden Materialien können das Verbundmaterial, beispielsweise CFK, selber sein, aber auch metallische Werkzeuge und Tools sowie andere Stoffe und Hilfsstoffe.

Eine Idee ist, die Leiter gegenüber störenden Einflüssen abzuschirmen und die Kapazität zwischen zwei Leitern, wobei diese von der Abschirmung umgeben sind zu messen. Eine weitere Idee enthält die Messung der Kapazität zwischen einem Leiter und einer diesen Leiter umgebenden Abschirmung.

Zwischen Leiter(n) und Abschirmung ist ein Hohlraum ausgebildet, in den das Harz eindringen kann. Dieser Hohlraum besteht aus Luft oder einem porösen/gewebeartigen Material, in das das Harz eindringen kann.

Die Abschirmung ist perforiert, damit das Harz eindringen kann. Der Durchmesser der Poren der Perforation ist so gewählt, dass das Harz gut eindringen kann, aber auch gute Abschirmung bei der entsprechenden Messfrequenz sichergestellt ist. Auch der Abstand zwischen Leiter(n) und Abschirmung ist entsprechend zu wählen.

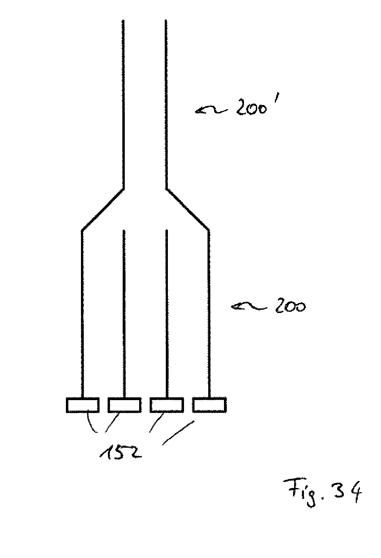

Mit den beschriebenen Sensoren kann bei Verwendung eines langen Sensors die Fließfront über eine größere Strecke hinweg erfasst werden. Die Messstrecke kann auch unterteilt werden. Um trotzdem nicht mehrere Sensoren handhaben und einbauen zu müssen, lässt sich eine Serienstruktur der in Fig. 34 schematisch dargestellten Art aufbauen. Dabei wird ein erster Teil der Messstrecke durch eine erste Sensoreinrichtung 200 und ein zweiter Teil der Messstrecke durch eine zweite Sensoreinrichtung 200' überwacht, die in einem gemeinsamen Bauteil oder einer gemeinsamen Schichtstruktur 66 integriert sind und auch an einem gemeinsamen Anschlussbereich angeschlossen werden können.

Der beschriebene Ansatz ist messtechnisch einfach, da lediglich die zeitliche Änderung der Kapazität erfasst wird. Deshalb ist auch eine drahtlose Messung möglich. Dazu kann der Sensor z.B. als Kapazität eines verstimmbaren Schwingkreises verwendet werden. Hierfür wird die Folie mit den Kontaktpads oberflächennah aus dem Bauteil bzw. aus dem Tooling-Aufbau herausgeführt und mit einer Antennenstruktur versehen.

Diese extrem dünnen Sensoren können nicht nur ins Werkzeug (Tooling) eingebracht oder in/auf Vakuumsäcke auf-/eingebracht, sondern sogar in das Laminat eingebracht werden.

Die beschriebenen Strukturen können bei einer weiteren Idee zur Bestimmung des Fließfrontverlaufs nicht nur bei einer kapazitiven Messung angewendet werden sondern auch bei der elektrischen Zeitbereichsreflektometrie (engl. electrical Time Domain Reflectometry, kurz: E-TDR).

Auch eine offene Struktur ist möglich, bei der nur auf einer Seite die Abschirmung angebracht ist. Die Abschirmungsfähigkeit ist dann zwar verringert, reicht jedoch für viele Anwendungen aus. Z.B. könnte der Sensor auf das metallische Werkzeug aufgelegt werden, welches auch als Abschirmung dienen kann.

Der Sensor lässt sich auch zur Bestimmung des Vernetzungsgrades verwenden. Denn mit zunehmender Aushärtung erhöht sich die Ionenviskosität des Harzes. Diese beeinflusst den Imaginärteil der relativen Dielektrizitätskonstante, welche bei verschiedenen Frequenzen gemessen werden kann. Die Ionenviskosität erhöht sich mit zunehmender Aushärtung asymptotisch. Es ist mit dem Sensor zur Fließfrontmessung eine Messung des Mittelwerts des Vernetzungsgrades möglich. Wenn der gemessene Mittelwert auch der maximal erreichbare Wert für die Ionenviskosität ist, kann davon ausgegangen werden, dass überall entlang des Fließfrontsensors das Harz ausgehärtet ist.

In den Sensor lassen sich zudem ein oder mehrere Temperatursensoren integrieren. Damit können von Temperaturunterschieden verursachte Messfehler (Einfluss auf Dielektrizität) kompensiert werden, aber auch die Temperatur als sehr wichtiger Prozessparameter erfasst werden. Die Integration in den Fließfront-Sensor vereinfacht Installation und Messung. Der Temperatursensor kann als Thermoelement realisiert werden oder noch einfacher als Widerstandsthermometer. Dazu kann im einfachsten Fall dasselbe Material und dieselbe Metallisierungsebene wie für die Leiterbahnen und/oder Abschirmungen zur Fließfrontmessung verwendet werden. Die Widerstandsmessung kann als Zwei- oder Vierleiter-Messung durchgeführt werden.

Die beschriebenen Prinzipien können nicht nur für Harz angewendet werden, sondern auch für andere Medien, wie beispielsweise Kleber.

Bezugszeichenliste:

| | |
|---|---|
| 10 | erste Leitung |
| 12 | zweite Leitung |
| 14 | Dielektrikum |
| 16 | Fasermaterial |
| 18 | Harz |
| 20 | Materialparameterermittlungsvorrichtung |
| 22 | Fasermaterial |
| 24 | Matrixmaterial |
| 26 | Verbundwerkstoff |
| 28 | Sensoreinrichtung |
| 30 | Innenelektrode |
| 34 | Außenelektrode |
| 38 | Probenraum |
| 40 | Dielektrikum |
| 42 | Steuereinrichtung |

| 44 | Spannungsimpulserzeugungsmodul |
|---|---|
| 46 | Detektionsspannungsimpuls |
| 48 | Spannungsimpulserfassungsmodul |
| 50 | Spannungsimpulslaufzeiterfassungsmodul |
| 52 | Auswertemodul |
| 56 | Spannungsimpulserzeugungsmodul |
| 58 | erster Detektionsspannungsimpuls |
| 60 | zweiter Detektionsspannungsimpuls |
| 62 | Spannungsimpulserfassungsmodul |
| 64 | Felderzeugungsanordnung |
| 66 | Schichtstruktur |
| 68 | erste elektrisch leitende Schicht |
| 70 | zweite elektrisch leitende Schicht |
| 72 | elektrisch isolierende Schicht |
| 76 | Isolierhülle |
| 78 | erste Leiterplatte |
| 80 | zweite Leiterplatte |
| 82 | erstes Schichtstrukturmodul |
| 84 | zweites Schichtstrukturmodul |
| 86 | äußerer Randbereich |
| 87 | Innenbereich |
| 87a | erster Innenbereich |
| 87b | zweiter Innenbereich |
| 88 | Durchkontaktierung |
| 90 | Leiterplattensubstrat |
| 92 | Infiltrationsbohrungen |
| 94 | Verbindungsanordnung |
| 96 | erstes Koaxialkabel |
| 98 | erster Innenleiter |
| 100 | erster Außenleiter |
| 102 | zweites Koaxialkabel |
| 104 | zweiter Innenleiter |
| 106 | zweiter Außenleiter |
| 108 | erste Verbindungseinrichtung |
| 110 | zweite Verbindungseinrichtung |
| 112 | Steckverbinder |
| 114 | Erdleiter |
| 120 | Sprungstelle |
| 122 | Permittivitätsübergang |
| 124 | Reflexionsspannungsimpuls |
| 126 | Transmissionsspannungsimpuls |
| 128 | Startsignal |
| 130 | Stoppsignal |
| 132 | erste Sprungstelle |
| 134 | zweite Sprungstelle |
| 136 | dritte Sprungstelle |
| 138 | Endabschnitt |
| 140 | erster Reflexionsspannungsimpuls |
| 142 | zweiter Reflexionsspannungsimpuls |
| 144 | erster Transmissionsspannungsimpuls |
| 148 | Startsignal |
| 150 | Stoppsignal |
| 152 | Anschlussflächen |
| 154 | Infiltatrionskanal |
| 156 | erste Bohrungen |
| 158 | zweite Bohrungen |
| 160 | Folie |
| 162 | Metallisierung auf einer Seite |

| | |
|---|---|
| 164 | Metallisierung auf der anderen Seite |
| 166 | Isolator |
| 168 | Infiltrationsöffnung |
| 170 | weitere Folie |
| 172 | Verbindungsschicht |
| 174 | Messschicht |
| 176 | Erdungsschicht |
| 200, 200' | Sensoreinrichtung |
| 202 | erste Innenelektrode |
| 204 | zweite Innenelektrode |
| 206 | Dielektrikum |
| 208 | Isoliermaterial |
| 210 | Außenelektrode |
| 214 | erste Sprungstelle |
| 216 | zweite Sprungstelle |
| 218 | dritte Sprungstelle |
| 220 | Permittivitätsübergang |

| | |
|---|---|
| $\varepsilon_1$ | erste Permittivität |
| $\varepsilon_2$ | zweite Permittivität |
| t | Spannungsimpulslaufzeit |
| $U_D$ | Detektionsspannungsimpulsamplitude |
| $U_{D1}$ | erste Detektionsspannungsimpulsamplitude |
| $U_{D2}$ | zweite Detektionsspannungsimpulsamplitude |
| $U_G$ | Gesamtreflexionsspannungsimpulsamplitude |
| $U_R$ | Reflexionsspannungsimpulsamplitude |
| $U_{R1}$ | erste Reflexionsspannungsimpulsamplitude |
| $U_{R2}$ | zweite Reflexionsspannungsimpulsamplitude |
| $U_T$ | Transmissionsspannungsimpulsamplitude |
| $U_{T1}$ | erste Transmissionsspannungsimpulsamplitude |
| $U_{T2}$ | zweite Transmissionsspannungsimpulsamplitude |

**Patentansprüche**

1. Sensoreinrichtung (28, 200, 200') zum Erfassen wenigstens eines Materialparameters eines ein Matrixmaterial (24) aufweisenden Verbundwerkstoffes, umfassend eine Schichtstruktur (66) mit einem darin vorgesehenen Probenraum (38) zum Aufnehmen des Matrixmaterials (24), mit einer durch elektrisch leitende Schichten (68, 70) gebildeten Felderzeugungsanordnung (64) zum Erzeugen eines elektrischen Feldes in dem Probenraum (38) und mit einer die elektrisch leitenden Schichten (68, 70) der Felderzeugungsanordnung (64) voneinander isolierenden Isolierschicht (72).

2. Sensoreinrichtung nach einem der vorstehenden Ansprüche, **dadurch gekennzeichnet, dass** die Schichtstruktur (66) eine den Probenraum (38) und/oder wenigstens einen Teil der Felderzeugungsanordnung (64) elektrisch abschirmende durch wenigstens eine elektrische leitende Schicht (70) gebildete Abschirmung aufweist.

3. Sensoreinrichtung nach einem der voranstehenden Ansprüche, **gekennzeichnet durch** wenigstens eines, mehrere oder alle der folgenden Merkmale:

   3.1 dass die Felderzeugungsanordnung (64) eine **durch** wenigstens eine elektrisch leitende Schicht gebildete erste Elektrode (30, 202) und eine **durch** wenigstens eine elektrisch leitende Schicht gebildete zweite Elektrode (34, 204) zum Erzeugen des elektrischen Feldes aufweist,
   3.2 dass die Felderzeugungsanordnung (64) wenigstens eine **durch** wenigstens eine elektrisch leitende Schicht der Schichtstruktur (66) gebildete Innenelektrode (30, 202, 204) und eine **durch** wenigstens eine elektrisch leitende Schicht der Schichtstruktur (66) gebildete die wenigstens eine Innenelektrode abschirmende Außenelektrode (34, 210) aufweist und/oder
   3.3 dass die Felderzeugungsanordnung (64) eine **durch** wenigstens eine elektrisch leitende Schicht der Schichtstruktur gebildete erste Innenelektrode (202) und eine **durch** wenigstens eine elektrische leitende Schicht gebildete zweite Innenelektrode (204) aufweist.

4. Sensoreinrichtung nach einem der vorstehenden Ansprüche, **dadurch gekennzeichnet, dass** die Sensoreinrichtung (28, 200, 200') sich flächenartig und/oder in einer Längsrichtung erstreckt und/oder ein Verhältnis von Länge zu Breite der Sensoreinrichtung von mindestens 5:1 aufweist.

5. Sensoreinrichtung nach einem der vorstehenden Ansprüche, **dadurch gekennzeichnet, dass** das die Schichtstruktur (66) eine mehrere oder alle der folgenden Schichten aufweist:

   5.1 eine elektrisch leitende Messschicht (174),
   5.2 eine elektrisch leitende Erdungsschicht (176) zum Anlegen an Masse,
   5.3 eine elektrisch leitende Zwischenerdungsschicht, die an derselben Seite der Isolierschicht wie die Messschicht (174) angeordnet ist,
   5.4 eine elektrisch isolierende Zwischenisolierschicht, die an die Isolierschicht und/oder die Messschicht und/oder die Zwischenerdungsschicht und/oder den Probenraum (38) angrenzt,
   5.5 eine elektrisch leitende Deckschicht, die an die Zwischenisolierschicht angrenzt, so dass Zwischenisolierschicht die Deckschicht von der Messschicht isoliert,
   5.6 eine Verbindungsschicht zum Verbinden von gesondert hergestellten Schichtstrukturmodulen, um so die Schichtstruktur zu bilden, und/oder
   5.7 eine Isolierschicht aus einem Lackmaterial, durch Spin-Coating aufgetragenem Material, aufgespraytem Material, durch Siebdruck aufgetragenem Material und/oder durch PVD aufgetragenem Material.

6. Sensoreinrichtung nach einem der vorstehenden Ansprüche, **dadurch gekennzeichnet, dass** sich die Felderzeugungsanordnung (64) über eine Großteil der Länge der Schichtstruktur (66) erstreckt.

7. Sensoreinrichtung nach einem der vorstehenden Ansprüche, **dadurch gekennzeichnet, dass** die Schichtstruktur (66) einen länglichen Durchlass oder eine Reihe von Durchlässen zum Durchlassen von zu messenden Material in den länglichen Probenraum (38) aufweist.

8. Sensoreinrichtung nach Anspruch 7, **dadurch gekennzeichnet, dass** eine Durchlassabmessung des wenigstens einen Durchlasses entsprechend einer Kapazitätsmessfrequenz ausgebildet ist und/oder dass der wenigstens eine Durchlass als eine sich im Wesentlichen in Längsrichtung erstreckende Öffnung oder dass eine Reihe von im Wesentlichen zylinderförmigen Öffnungen vorgesehen sind und/oder dass der Durchlass auf lediglich einer Seite der Schichtstruktur (66) vorgesehen ist.

9. Sensoreinrichtung nach einem der vorstehenden Ansprüche, **dadurch gekennzeichnet, dass** die Schichtstruktur (66) spiegelsymmetrisch bezüglich einer Längsmittelebene oder bezüglich zwei Längsmittelebenen ausgebildet ist.

10. Messvorrichtung zum Messen wenigstens eines Materialparameters eines ein Matrixmaterial aufweisenden Verbundwerkstoffes entlang einer Messstrecke, mit einem sich entlang der Messstrecke erstreckenden Bauteil, an dem mehrere Sensoreinrichtungen (28, 200, 200')nach einem der voranstehenden Ansprüche an zumindest teilweise unterschiedlichen Bereichen der Messstrecke ausgebildet sind.

11. Messvorrichtung nach Anspruch 10, **gekennzeichnet durch** eine Schnittstelle, die zum gemeinsamen elektrischen und/oder drahtlosen Anschließen von wenigstens zwei der mehreren Sensoreinrichtungen (28, 200, 200') an eine Materialparametererfassungsvorrichtung ausgebildet ist.

12. Verfahren zum Herstellen eines Sensoreinrichtung (28, 200, 200') nach einem der vorstehenden Ansprüche mit den Schritten:

   a) Bereitstellen wenigstens eines Schichtstrukturrohlings mit einer elektrisch isolierenden Isolierschicht und mit einer elektrisch leitenden Schicht und
   b) Ausbilden von Elektroden einer Felderzeugungsanordnung (64) und/oder einer Abschirmung und/oder des Probenraums (38) durch Materialstrukturierung, Materialauftrag und/oder Materialabtrag an dem wenigstens einen Schichtstrukturrohling.

13. Verfahren nach Anspruch 12,
    **gekennzeichnet durch**

c) Bilden von mehreren Schichtstrukturmodulen (82, 84) aus mehreren Schichtstrukturrohlingen und
d) Verbinden der Schichtstrukturmodule (82, 84) zu einer den Probenraum (38) und die Felderzeugungsanordnung (64) und eine Abschirmung aufweisenden Schichtstruktur (66).

14. Verwendung einer Sensoreinrichtung (28, 200, 200') nach einem der Ansprüche 1 bis 9 zum Messen einer Fließfront und/oder eines Aushärtungsgrades und/oder eines Vernetzungsgrades beim Herstellen von faserverstärkten Verbundbauteilen.

15. Verwendung nach Anspruch 14, **dadurch gekennzeichnet, dass** die Sensoreinrichtung (28, 200, 200') in ein Werkzeug und/oder in einem Vakuumsack und/oder auf einem Vakuumsack und/oder in dem Verbundbauteil eingebracht ist.

Fig. 1

Fig. 2

EP 3 035 043 A1

Fig. 3

Fig. 4

21

Fig. 5

Fig. 6

Fig. 7

Fig. 8

Fig. 9A

Fig. 9 B

Fig. 9C

Fig. 10A

Fig. 10B

Fig. 10C

Fig. 11

Fig. 12

Fig. 13
C-C

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 22

Fig. 28

Fig. 29

Fig. 30

Fig. 31

Fig. 32

Fig. 33

~ 200'

~ 200

152

Fig. 34

Fig. 35

Stand der Technik

**Europäisches Patentamt**
**European Patent Office**
**Office européen des brevets**

# EUROPÄISCHER RECHERCHENBERICHT

Nummer der Anmeldung

EP 14 19 8966

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/027137 A1 (SHERRARD WAYNE [CA]) 12. Februar 2004 (2004-02-12) * Absätze [0001], [0002], [0007], [0019], [0020], [0025], [0028]; Abbildungen 1,2 * | 1,2, 10-15 | INV. G01N27/22 G01F23/26 |
| X | EP 2 012 098 A1 (SICK AG [DE]) 7. Januar 2009 (2009-01-07) * Absätze [0042] - [0046]; Abbildungen 7,8 * | 1-15 | |
| X | US 4 786 857 A (MOHR CHARLES L [US] ET AL) 22. November 1988 (1988-11-22) * Zusammenfassung; Abbildungen 2,3 * | 1-15 | |
| X | GB 2 364 777 A (SONDEX LTD [GB]) 6. Februar 2002 (2002-02-06) * Seite 5, Zeile 6 - Seite 6, Zeile 8; Abbildung 2 * | 1-15 | |
| X | WO 2006/029427 A1 (EXESS ENGINEERING GES M B H [AT]; STUHLBACHER FRANZ [AT]) 23. März 2006 (2006-03-23) * Seite 4, Zeile 6 - Seite 5, Zeile 8; Abbildung 1 * | 1-15 | RECHERCHIERTE SACHGEBIETE (IPC) G01N G01F |
| X | US 2011/079522 A1 (WEBSTER GRAEME [GB] ET AL) 7. April 2011 (2011-04-07) * Absätze [0019], [0020], [0024], [0026] - [0030]; Abbildung 1 * | 1,3-5,7, 12,13 | |

-/--

Der vorliegende Recherchenbericht wurde für alle Patentansprüche erstellt

| Recherchenort | Abschlußdatum der Recherche | Prüfer |
|---|---|---|
| München | 22. Mai 2015 | Stussi, Elisa |

Europäisches
Patentamt

European
Patent Office

Office européen
des brevets

**EUROPÄISCHER RECHERCHENBERICHT**

Nummer der Anmeldung

EP 14 19 8966

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | GAURAV PANDEY ET AL: "Smart tooling with integrated time domain reflectometry sensing line for non-invasive flow and cure monitoring during composites manufacturing", COMPOSITES PART A: APPLIED SCIENCE AND MANUFACTURING, Bd. 47, 15. Dezember 2012 (2012-12-15), Seiten 102-108, XP055190729, ISSN: 1359-835X, DOI: 10.1016/j.compositesa.2012.11.017 * das ganze Dokument * ----- | 1-15 | |
| A | "Time-domain reflectometry", , 1. Januar 2004 (2004-01-01), XP055190874, Gefunden im Internet: URL:http://udspace.udel.edu/bitstream/handle/19716/80/TDR.pdf?sequence=1 [gefunden am 2015-05-21] * das ganze Dokument * ----- | 1-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 22. Mai 2015 | Stussi, Elisa |

**ANHANG ZUM EUROPÄISCHEN RECHERCHENBERICHT
ÜBER DIE EUROPÄISCHE PATENTANMELDUNG NR.**

EP 14 19 8966

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-05-2015

| Im Recherchenbericht angeführtes Patentdokument | Datum der Veröffentlichung | Mitglied(er) der Patentfamilie | Datum der Veröffentlichung |
|---|---|---|---|
| US 2004027137 A1 | 12-02-2004 | CA 2384257 A1<br>US 2004027137 A1 | 29-10-2003<br>12-02-2004 |
| EP 2012098 A1 | 07-01-2009 | DE 102007030847 A1<br>DE 202008018156 U1<br>EP 2012098 A1 | 15-01-2009<br>22-12-2011<br>07-01-2009 |
| US 4786857 A | 22-11-1988 | DE 3788590 D1<br>DE 3788590 T2<br>EP 0244033 A2<br>US 4786857 A | 10-02-1994<br>14-07-1994<br>04-11-1987<br>22-11-1988 |
| GB 2364777 A | 06-02-2002 | KEINE | |
| WO 2006029427 A1 | 23-03-2006 | EP 1834159 A1<br>WO 2006029427 A1 | 19-09-2007<br>23-03-2006 |
| US 2011079522 A1 | 07-04-2011 | AU 2010224341 A1<br>BR PI1003687 A2<br>CA 2716259 A1<br>CN 102033087 A<br>EP 2308991 A1<br>ES 2428744 T3<br>HK 1155493 A1<br>JP 2011080990 A<br>KR 20110036674 A<br>SG 169971 A1<br>TW 201140045 A<br>US 2011079522 A1 | 01-03-2012<br>29-01-2013<br>02-04-2011<br>27-04-2011<br>13-04-2011<br>11-11-2013<br>07-03-2014<br>21-04-2011<br>08-04-2011<br>29-04-2011<br>16-11-2011<br>07-04-2011 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SERNEK ; KAMKE.** *Int. J. Adhes. Adhes.,* 2007, vol. 27 (7), 562-567
- **KAZILAS ; PARTRIDGE.** *Poymer,* vol. 46 (16), 5868-5878
- **HARDIS et al.** *Compos. Part Appl. Sci. Manuf.,* 2013, vol. 49, 100-108
- **DOMINAUSKAS et al.** *Compos. Part Appl. Sci. Manuf.,* 2003, vol. 34, 67-74
- *COMPOS. PART APP. SCI. MANUF.,* 2007, vol. 38, 138-146